# EUROPEAN PATENT SPECIFICATION

(11) **EP 2 286 232 B1**
(45) Date of publication and mention of the grant of the patent: **29.02.2012**
(21) Application number: 09745563.8
(22) Date of filing: 14.05.2009
(51) Int. Cl.: G01N 33/573

(54) **METHODS FOR THE IDENTIFICATION OF PARP INTERACTING MOLECULES AND FOR PURIFICATION OF PARP PROTEINS**
VERFAHREN ZUR IDENTIFIZIERUNG VON MIT PARP WECHSELWIRKENDEN MOLEKÜLEN UND ZUR AUFREINIGUNG VON PARP-PROTEINEN
PROCÉDÉS POUR L'IDENTIFICATION DE MOLÉCULES ENTRANT EN INTERACTION AVEC PARP ET POUR LA PURIFICATION DE PROTÉINES PARP

(30) Priority: 16.05.2008 EP 08009128
(43) Date of publication of application: 23.02.2011
(73) Proprietor: CELLZOME AG, 69117 Heidelberg (DE)
(72) Inventor: DREWES, Gerard, 69121 Heidelberg (DE); READER, Valerie, Linton Cambridgeshire CB21 4HU (GB)
(74) Representative: Lahrtz, Fritz
(86) International application number: PCT/EP2009/003426
(87) International publication number: WO 2009/138229

(56) References cited:
- WO-A-03/080610
- WO-A-2005/011700
- WO-A-2005/046678
- WO-A-2007/058850
- DAUB H ET AL: "EVALUATION OF KINASE INHIBITOR SELECTIVITY BY CHEMICAL PROTEOMICS" ASSAY AND DRUG DEVELOPMENT TECHNOLOGIES, vol. 2, no. 2, 2004, pages 215-224, XP001183642
- D'AMOURS DAMIEN ET AL: "Purification of the death substrate poly(ADP-ribose) polymerase" ANALYTICAL BIOCHEMISTRY, vol. 249, no. 1, 1997, pages 106-108, XP002501125 cited in the application

### SEQUENCE LISTING

<110> Cellzome AG
<120> Methods for the Identification of PARP Interacting Molecules and for the Purification of PARP Proteins
<130> CEL65370PC
<160> 5
<170> PatentIn version 3.5
<210> 1
   <211> 1014
   <212> PRT
   <213> Homo sapiens
<400> 1
   Met Ala Glu Ser Ser Asp Lys Leu Tyr Arg Val Glu Tyr Ala Lys Ser
   1               5                   10                  15
   Gly Arg Ala Ser Cys Lys Lys Cys Ser Glu Ser Ile Pro Lys Asp Ser
               20                  25                  30
   Leu Arg Met Ala Ile Met Val Gln Ser Pro Met Phe Asp Gly Lys Val
           35                  40                  45
   Pro His Trp Tyr His Phe Ser Cys Phe Trp Lys Val Gly His Ser Ile
       50                  55                  60
   Arg His Pro Asp Val Glu Val Asp Gly Phe Ser Glu Leu Arg Trp Asp
   65                  70                  75                  80
   Asp Gln Gln Lys Val Lys Lys Thr Ala Glu Ala Gly Gly Val Thr Gly
                   85                  90                  95
   Lys Gly Gln Asp Gly Ile Gly Ser Lys Ala Glu Lys Thr Leu Gly Asp
               100                 105                 110
   Phe Ala Ala Glu Tyr Ala Lys Ser Asn Arg Ser Thr Cys Lys Gly Cys
           115                 120                 125
   Met Glu Lys Ile Glu Lys Gly Gln Val Arg Leu Ser Lys Lys Met Val
       130                 135                 140
   Asp Pro Glu Lys Pro Gln Leu Gly Met Ile Asp Arg Trp Tyr His Pro
   145                 150                 155                 160
   Gly Cys Phe Val Lys Asn Arg Glu Glu Leu Gly Phe Arg Pro Glu Tyr
                   165                 170                 175
   Ser Ala Ser Gln Leu Lys Gly Phe Ser Leu Leu Ala Thr Glu Asp Lys
               180                 185                 190
   Glu Ala Leu Lys Lys Gln Leu Pro Gly Val Lys Ser Glu Gly Lys Arg
   195 200 205
   Lys Gly Asp Glu Val Asp Gly Val Asp Glu Val Ala Lys Lys Lys Ser
   210 215 220
   Lys Lys Glu Lys Asp Lys Asp Ser Lys Leu Glu Lys Ala Leu Lys Ala
   225 230 235 240
   Gln Asn Asp Leu Ile Trp Asn Ile Lys Asp Glu Leu Lys Lys Val Cys
   245 250 255
   Ser Thr Asn Asp Leu Lys Glu Leu Leu Ile Phe Asn Lys Gln Gln Val
   260 265 270
   Pro Ser Gly Glu Ser Ala Ile Leu Asp Arg Val Ala Asp Gly Met Val
   275 280 285
   Phe Gly Ala Leu Leu Pro Cys Glu Glu Cys Ser Gly Gln Leu Val Phe
   290 295 300
   Lys Ser Asp Ala Tyr Tyr Cys Thr Gly Asp Val Thr Ala Trp Thr Lys
   305 310 315 320
   Cys Met Val Lys Thr Gln Thr Pro Asn Arg Lys Glu Trp Val Thr Pro
   325 330 335
   Lys Glu Phe Arg Glu Ile Ser Tyr Leu Lys Lys Leu Lys Val Lys Lys
   340 345 350
   Gln Asp Arg Ile Phe Pro Pro Glu Thr Ser Ala Ser Val Ala Ala Thr
   355 360 365
   Pro Pro Pro Ser Thr Ala Ser Ala Pro Ala Ala Val Asn Ser Ser Ala
   370 375 380
   Ser Ala Asp Lys Pro Leu Ser Asn Met Lys Ile Leu Thr Leu Gly Lys
   385 390 395 400
   Leu Ser Arg Asn Lys Asp Glu Val Lys Ala Met Ile Glu Lys Leu Gly
   405 410 415
   Gly Lys Leu Thr Gly Thr Ala Asn Lys Ala Ser Leu Cys Ile Ser Thr
   420 425 430
   Lys Lys Glu Val Glu Lys Met Asn Lys Lys Met Glu Glu Val Lys Glu
   435 440 445
   Ala Asn Ile Arg Val Val Ser Glu Asp Phe Leu Gln Asp Val Ser Ala
   450 455 460
   Ser Thr Lys Ser Leu Gln Glu Leu Phe Leu Ala His Ile Leu Ser Pro
   465                 470                 475                 480
   Trp Gly Ala Glu Val Lys Ala Glu Pro Val Glu Val Val Ala Pro Arg
                   485                 490                 495
   Gly Lys Ser Gly Ala Ala Leu Ser Lys Lys Ser Lys Gly Gln Val Lys
               500                 505                 510
   Glu Glu Gly Ile Asn Lys Ser Glu Lys Arg Met Lys Leu Thr Leu Lys
           515                 520                 525
   Gly Gly Ala Ala Val Asp Pro Asp Ser Gly Leu Glu His Ser Ala His
       530                 535                 540
   Val Leu Glu Lys Gly Gly Lys Val Phe Ser Ala Thr Leu Gly Leu Val
   545                 550                 555                 560
   Asp Ile Val Lys Gly Thr Asn Ser Tyr Tyr Lys Leu Gln Leu Leu Glu
                   565                 570                 575
   Asp Asp Lys Glu Asn Arg Tyr Trp Ile Phe Arg Ser Trp Gly Arg Val
               580                 585                 590
   Gly Thr Val Ile Gly Ser Asn Lys Leu Glu Gln Met Pro Ser Lys Glu
           595                 600                 605
   Asp Ala Ile Glu His Phe Met Lys Leu Tyr Glu Glu Lys Thr Gly Asn
       610                 615                 620
   Ala Trp His Ser Lys Asn Phe Thr Lys Tyr Pro Lys Lys Phe Tyr Pro
   625                 630                 635                 640
   Leu Glu Ile Asp Tyr Gly Gln Asp Glu Glu Ala Val Lys Lys Leu Thr
                   645                 650                 655
   Val Asn Pro Gly Thr Lys Ser Lys Leu Pro Lys Pro Val Gln Asp Leu
               660                 665                 670
   Ile Lys Met Ile Phe Asp Val Glu Ser Met Lys Lys Ala Met Val Glu
           675                 680                 685
   Tyr Glu Ile Asp Leu Gln Lys Met Pro Leu Gly Lys Leu Ser Lys Arg
       690                 695                 700
   Gln Ile Gln Ala Ala Tyr Ser Ile Leu Ser Glu Val Gln Gln Ala Val
   705                 710                 715                 720
   Ser Gln Gly Ser Ser Asp Ser Gln Ile Leu Asp Leu Ser Asn Arg Phe
                   725                 730                 735
   Tyr Thr Leu Ile Pro His Asp Phe Gly Met Lys Lys Pro Pro Leu Leu
   740 745 750
   Asn Asn Ala Asp Ser Val Gln Ala Lys Val Glu Met Leu Asp Asn Leu
   755 760 765
   Leu Asp Ile Glu Val Ala Tyr Ser Leu Leu Arg Gly Gly Ser Asp Asp
   770 775 780
   Ser Ser Lys Asp Pro Ile Asp Val Asn Tyr Glu Lys Leu Lys Thr Asp
   785 790 795 800
   Ile Lys Val Val Asp Arg Asp Ser Glu Glu Ala Glu Ile Ile Arg Lys
   805 810 815
   Tyr Val Lys Asn Thr His Ala Thr Thr His Asn Ala Tyr Asp Leu Glu
   820 825 830
   Val Ile Asp Ile Phe Lys Ile Glu Arg Glu Gly Glu Cys Gln Arg Tyr
   835 840 845
   Lys Pro Phe Lys Gln Leu His Asn Arg Arg Leu Leu Trp His Gly Ser
   850 855 860
   Arg Thr Thr Asn Phe Ala Gly Ile Leu Ser Gln Gly Leu Arg Ile Ala
   865 870 875 880
   Pro Pro Glu Ala Pro Val Thr Gly Tyr Met Phe Gly Lys Gly Ile Tyr
   885 890 895
   Phe Ala Asp Met Val Ser Lys Ser Ala Asn Tyr Cys His Thr Ser Gln
   900 905 910
   Gly Asp Pro Ile Gly Leu Ile Leu Leu Gly Glu Val Ala Leu Gly Asn
   915 920 925
   Met Tyr Glu Leu Lys His Ala Ser His Ile Ser Lys Leu Pro Lys Gly
   930 935 940
   Lys His Ser Val Lys Gly Leu Gly Lys Thr Thr Pro Asp Pro Ser Ala
   945 950 955 960
   Asn Ile Ser Leu Asp Gly Val Asp Val Pro Leu Gly Thr Gly Ile Ser
   965 970 975
   Ser Gly Val Asn Asp Thr Ser Leu Leu Tyr Asn Glu Tyr Ile Val Tyr
   980 985 990
   Asp Ile Ala Gln Val Asn Leu Lys Tyr Leu Leu Lys Leu Lys Phe Asn
   995 1000 1005
   Phe Lys Thr Ser Leu Trp
   1010
<210> 2
   <211> 1025
   <212> PRT
   <213> Homo sapiens
<400> 2
   Met Val Ala Met Ala Glu Ala Glu Ala Gly Val Ala Val Glu Val Arg
   1               5                   10                  15
   Gly Leu Pro Pro Ala Val Pro Asp Glu Leu Leu Thr Leu Tyr Phe Glu
               20                  25                  30
   Asn Arg Arg Arg Ser Gly Gly Gly Pro Val Leu Ser Trp Gln Arg Leu
           35                  40                  45
   Gly Cys Gly Gly Val Leu Thr Phe Arg Glu Pro Ala Asp Ala Glu Arg
       50                  55                  60
   Val Leu Ala Gln Ala Asp His Glu Leu His Gly Ala Gln Leu Ser Leu
   65                  70                  75                  80
   Arg Pro Ala Pro Pro Arg Ala Pro Ala Arg Leu Leu Leu Gln Gly Leu
                   85                  90                  95
   Pro Pro Gly Thr Thr Pro Gln Arg Leu Glu Gln His Val Gln Ala Leu
               100                 105                 110
   Leu Arg Ala Ser Gly Leu Pro Val Gln Pro Cys Cys Ala Leu Ala Ser
           115                 120                 125
   Pro Arg Pro Asp Arg Ala Leu Val Gln Leu Pro Lys Pro Leu Ser Glu
       130                 135                 140
   Ala Asp Val Arg Val Leu Glu Glu Gln Ala Gln Asn Leu Gly Leu Glu
   145                 150                 155                 160
   Gly Thr Leu Val Ser Leu Ala Arg Val Pro Gln Ala Arg Ala Val Arg
                   165                 170                 175
   Val Val Gly Asp Gly Ala Ser Val Asp Leu Leu Leu Leu Glu Leu Tyr
               180                 185                 190
   Leu Glu Asn Glu Arg Arg Ser Gly Gly Gly Pro Leu Glu Asp Leu Gln
           195                 200                 205
   Arg Leu Pro Gly Pro Leu Gly Thr Val Ala Ser Phe Gln Gln Trp Gln
       210                 215                 220
   Val Ala Glu Arg Val Leu Gln Gln Glu His Arg Leu Gln Gly Ser Glu
   225 230 235 240
   Leu Ser Leu Val Pro His Tyr Asp Ile Leu Glu Pro Glu Glu Leu Ala
   245 250 255
   Glu Asn Thr Ser Gly Gly Asp His Pro Ser Thr Gln Gly Pro Arg Ala
   260 265 270
   Thr Lys His Ala Leu Leu Arg Thr Gly Gly Leu Val Thr Ala Leu Gln
   275 280 285
   Gly Ala Gly Thr Val Thr Met Gly Ser Gly Glu Glu Pro Gly Gln Ser
   290 295 300
   Gly Ala Ser Leu Arg Thr Gly Pro Met Val Gln Gly Arg Gly Ile Met
   305 310 315 320
   Thr Thr Gly Ser Gly Gln Glu Pro Gly Gln Ser Gly Thr Ser Leu Arg
   325 330 335
   Thr Gly Pro Met Gly Ser Leu Gly Gln Ala Glu Gln Val Ser Ser Met
   340 345 350
   Pro Met Gly Ser Leu Glu His Glu Gly Leu Val Ser Leu Arg Pro Val
   355 360 365
   Gly Leu Gln Glu Gln Glu Gly Pro Met Ser Leu Gly Pro Val Gly Ser
   370 375 380
   Ala Gly Pro Val Glu Thr Ser Lys Gly Leu Leu Gly Gln Glu Gly Leu
   385 390 395 400
   Val Glu Ile Ala Met Asp Ser Pro Glu Gln Glu Gly Leu Val Gly Pro
   405 410 415
   Met Glu Ile Thr Met Gly Ser Leu Glu Lys Ala Gly Pro Val Ser Pro
   420 425 430
   Gly Cys Val Lys Leu Ala Gly Gln Glu Gly Leu Val Glu Met Val Leu
   435 440 445
   Leu Met Glu Pro Gly Ala Met Arg Phe Leu Gln Leu Tyr His Glu Asp
   450 455 460
   Leu Leu Ala Gly Leu Gly Asp Val Ala Leu Leu Pro Leu Glu Gly Pro
   465 470 475 480
   Asp Met Thr Gly Phe Arg Leu Cys Gly Ala Gln Ala Ser Cys Gln Ala
   485 490 495
   Ala Glu Glu Phe Leu Arg Ser Leu Leu Gly Ser Ile Ser Cys His Val
               500                 505                 510
   Leu Cys Leu Glu His Pro Gly Ser Ala Arg Phe Leu Leu Gly Pro Glu
           515                 520                 525
   Gly Gln His Leu Leu Gln Gly Leu Glu Ala Gln Phe Gln Cys Val Phe
       530                 535                 540
   Gly Thr Glu Arg Leu Ala Thr Ala Thr Leu Asp Thr Gly Leu Glu Glu
   545                 550                 555                 560
   Val Asp Pro Thr Glu Ala Leu Pro Val Leu Pro Gly Asn Ala His Thr
                   565                 570                 575
   Leu Trp Thr Pro Asp Ser Thr Gly Gly Asp Gln Glu Asp Val Ser Leu
               580                 585                 590
   Glu Glu Val Arg Glu Leu Leu Ala Thr Leu Glu Gly Leu Asp Leu Asp
           595                 600                 605
   Gly Glu Asp Trp Leu Pro Arg Glu Leu Glu Glu Glu Gly Pro Gln Glu
       610                 615                 620
   Gln Pro Glu Glu Glu Val Thr Pro Gly His Glu Glu Glu Glu Pro Val
   625                 630                 635                 640
   Ala Pro Ser Thr Val Ala Pro Arg Trp Leu Glu Glu Glu Ala Ala Leu
                   645                 650                 655
   Gln Leu Ala Leu His Arg Ser Leu Glu Pro Gln Gly Gln Val Ala Glu
               660                 665                 670
   Gln Glu Glu Ala Ala Ala Leu Arg Gln Ala Leu Thr Leu Ser Leu Leu
           675                 680                 685
   Glu Gln Pro Pro Leu Glu Ala Glu Glu Pro Pro Asp Gly Gly Thr Asp
       690                 695                 700
   Gly Lys Ala Gln Leu Val Val His Ser Ala Phe Glu Gln Asp Val Glu
   705                 710                 715                 720
   Glu Leu Asp Arg Ala Leu Arg Ala Ala Leu Glu Val His Val Gln Glu
                   725                 730                 735
   Glu Thr Val Gly Pro Trp Arg Arg Thr Leu Pro Ala Glu Leu Arg Ala
               740                 745                 750
   Arg Leu Glu Arg Cys His Gly Val Ser Val Ala Leu Arg Gly Asp Cys
           755                 760                 765
   Thr Ile Leu Arg Gly Phe Gly Ala His Pro Ala Arg Ala Ala Arg His
   770 775 780
   Leu Val Ala Leu Leu Ala Gly Pro Trp Asp Gln Ser Leu Ala Phe Pro
   785 790 795 800
   Leu Ala Ala Ser Gly Pro Thr Leu Ala Gly Gln Thr Leu Lys Gly Pro
   805 810 815
   Trp Asn Asn Leu Glu Arg Leu Ala Glu Asn Thr Gly Glu Phe Gln Glu
   820 825 830
   Val Val Arg Ala Phe Tyr Asp Thr Leu Asp Ala Ala Arg Ser Ser Ile
   835 840 845
   Arg Val Val Arg Val Glu Arg Val Ser His Pro Leu Leu Gln Gln Gln
   850 855 860
   Tyr Glu Leu Tyr Arg Glu Arg Leu Leu Gln Arg Cys Glu Arg Arg Pro
   865 870 875 880
   Val Glu Gln Val Leu Tyr His Gly Thr Thr Ala Pro Ala Val Pro Asp
   885 890 895
   Ile Cys Ala His Gly Phe Asn Arg Ser Phe Cys Gly Arg Asn Ala Thr
   900 905 910
   Val Tyr Gly Lys Gly Val Tyr Phe Ala Arg Arg Ala Ser Leu Ser Val
   915 920 925
   Gln Asp Arg Tyr Ser Pro Pro Asn Ala Asp Gly His Lys Ala Val Phe
   930 935 940
   Val Ala Arg Val Leu Thr Gly Asp Tyr Gly Gln Gly Arg Arg Gly Leu
   945 950 955 960
   Arg Ala Pro Pro Leu Arg Gly Pro Gly His Val Leu Leu Arg Tyr Asp
   965 970 975
   Ser Ala Val Asp Cys Ile Cys Gln Pro Ser Ile Phe Val Ile Phe His
   980 985 990
   Asp Thr Gln Ala Leu Pro Thr His Leu Ile Thr Cys Glu His Val Pro
   995 1000 1005
   Arg Ala Ser Pro Asp Asp Pro Ser Gly Leu Pro Gly Arg Ser Pro
   1010 1015 1020
   Asp Thr
   1025
<210> 3
   <211> 1638
   <212> PRT
   <213> Homo sapiens
<400> 3
   Met Leu Ile Leu Leu Val Glu Asn Ile Ser Gly Leu Ser Asn Asp Asp
   1               5                   10                  15
   Phe Gln Val Glu Ile Ile Arg Asp Phe Asp Val Ala Val Val Thr Phe
               20                  25                  30
   Gln Lys His Ile Asp Thr Ile Arg Phe Val Asp Asp Cys Thr Lys His
           35                  40                  45
   His Ser Ile Lys Gln Leu Gln Leu Ser Pro Arg Leu Leu Glu Val Thr
       50                  55                  60
   Asn Thr Ile Arg Val Glu Asn Leu Pro Pro Gly Ala Asp Asp Tyr Ser
   65                  70                  75                  80
   Leu Lys Leu Phe Phe Glu Asn Pro Tyr Asn Gly Gly Gly Arg Val Ala
                   85                  90                  95
   Asn Val Glu Tyr Phe Pro Glu Glu Ser Ser Ala Leu Ile Glu Phe Phe
               100                 105                 110
   Asp Arg Lys Val Leu Asp Thr Ile Met Ala Thr Lys Leu Asp Phe Asn
           115                 120                 125
   Lys Met Pro Leu Ser Val Phe Pro Tyr Tyr Ala Ser Leu Gly Thr Ala
       130                 135                 140
   Leu Tyr Gly Lys Glu Lys Pro Leu Ile Lys Leu Pro Ala Pro Phe Glu
   145                 150                 155                 160
   Glu Ser Leu Asp Leu Pro Leu Trp Lys Phe Leu Gln Lys Lys Asn His
                   165                 170                 175
   Leu Ile Glu Glu Ile Asn Asp Glu Met Arg Arg Cys His Cys Glu Leu
               180                 185                 190
   Thr Trp Ser Gln Leu Ser Gly Lys Val Thr Ile Arg Pro Ala Ala Thr
           195                 200                 205
   Leu Val Asn Glu Gly Arg Pro Arg Ile Lys Thr Trp Gln Ala Asp Thr
       210                 215                 220
   Ser Thr Thr Leu Ser Ser Ile Arg Ser Lys Tyr Lys Val Asn Pro Ile
   225                 230                 235                 240
   Lys Val Asp Pro Thr Met Trp Asp Thr Ile Lys Asn Asp Val Lys Asp
                   245                 250                 255
   Asp Arg Ile Leu Ile Glu Phe Asp Thr Leu Lys Glu Met Val Ile Leu
               260                 265                 270
   Ala Gly Lys Ser Glu Asp Val Gln Ser Ile Glu Val Gln Val Arg Glu
           275                 280                 285
   Leu Ile Glu Ser Thr Thr Gln Lys Ile Lys Arg Glu Glu Gln Ser Leu
       290                 295                 300
   Lys Glu Lys Met Ile Ile Ser Pro Gly Arg Tyr Phe Leu Leu Cys His
   305                 310                 315                 320
   Ser Ser Leu Leu Asp His Leu Leu Thr Glu Cys Pro Glu Ile Glu Ile
                   325                 330                 335
   Cys Tyr Asp Arg Val Thr Gln His Leu Cys Leu Lys Gly Pro Ser Ala
               340                 345                 350
   Asp Val Tyr Lys Ala Lys Cys Glu Ile Gln Glu Lys Val Tyr Thr Met
           355                 360                 365
   Ala Gln Lys Asn Ile Gln Val Ser Pro Glu Ile Phe Gln Phe Leu Gln
       370                 375                 380
   Gln Val Asn Trp Lys Glu Phe Ser Lys Cys Leu Phe Ile Ala Gln Lys
   385                 390                 395                 400
   Ile Leu Ala Leu Tyr Glu Leu Glu Gly Thr Thr Val Leu Leu Thr Ser
                   405                 410                 415
   Cys Ser Ser Glu Ala Leu Leu Glu Ala Glu Lys Gln Met Leu Ser Ala
               420                 425                 430
   Leu Asn Tyr Lys Arg Ile Glu Val Glu Asn Lys Glu Val Leu His Gly
           435                 440                 445
   Lys Lys Trp Lys Gly Leu Thr His Asn Leu Leu Lys Lys Gln Asn Ser
       450                 455                 460
   Ser Pro Asn Thr Val Ile Ile Asn Glu Leu Thr Ser Glu Thr Thr Ala
   465                 470                 475                 480
   Glu Val Ile Ile Thr Gly Cys Val Lys Glu Val Asn Glu Thr Tyr Lys
                   485                 490                 495
   Leu Leu Phe Asn Phe Val Glu Gln Asn Met Lys Ile Glu Arg Leu Val
               500                 505                 510
   Glu Val Lys Pro Ser Leu Val Ile Asp Tyr Leu Lys Thr Glu Lys Lys
           515                 520                 525
   Leu Phe Trp Pro Lys Ile Lys Lys Val Asn Val Gln Val Ser Phe Asn
       530                 535                 540
   Pro Glu Asn Lys Gln Lys Gly Ile Leu Leu Thr Gly Ser Lys Thr Glu
   545                 550                 555                 560
   Val Leu Lys Ala Val Asp Ile Val Lys Gln Val Trp Asp Ser Val Cys
                   565                 570                 575
   Val Lys Ser Val His Thr Asp Lys Pro Gly Ala Lys Gln Phe Phe Gln
               580                 585                 590
   Asp Lys Ala Arg Phe Tyr Gln Ser Glu Ile Lys Arg Leu Phe Gly Cys
           595                 600                 605
   Tyr Ile Glu Leu Gln Glu Asn Glu Val Met Lys Glu Gly Gly Ser Pro
       610                 615                 620
   Ala Gly Gln Lys Cys Phe Ser Arg Thr Val Leu Ala Pro Gly Val Val
   625                 630                 635                 640
   Leu Ile Val Gln Gln Gly Asp Leu Ala Arg Leu Pro Val Asp Val Val
                   645                 650                 655
   Val Asn Ala Ser Asn Glu Asp Leu Lys His Tyr Gly Gly Leu Ala Ala
               660                 665                 670
   Ala Leu Ser Lys Ala Ala Gly Pro Glu Leu Gln Ala Asp Cys Asp Gln
           675                 680                 685
   Ile Val Lys Arg Glu Gly Arg Leu Leu Pro Gly Asn Ala Thr Ile Ser
       690                 695                 700
   Lys Ala Gly Lys Leu Pro Tyr His His Val Ile His Ala Val Gly Pro
   705                 710                 715                 720
   Arg Trp Ser Gly Tyr Glu Ala Pro Arg Cys Val Tyr Leu Leu Arg Arg
                   725                 730                 735
   Ala Val Gln Leu Ser Leu Cys Leu Ala Glu Lys Tyr Lys Tyr Arg Ser
               740                 745                 750
   Ile Ala Ile Pro Ala Ile Ser Ser Gly Val Phe Gly Phe Pro Leu Gly
           755                 760                 765
   Arg Cys Val Glu Thr Ile Val Ser Ala Ile Lys Glu Asn Phe Gln Phe
       770                 775                 780
   Lys Lys Asp Gly His Cys Leu Lys Glu Ile Tyr Leu Val Asp Val Ser
   785                 790                 795                 800
   Glu Lys Thr Val Glu Ala Phe Ala Glu Ala Val Lys Thr Val Phe Lys
                   805                 810                 815
   Ala Thr Leu Pro Asp Thr Ala Ala Pro Pro Gly Leu Pro Pro Ala Ala
               820                 825                 830
   Ala Gly Pro Gly Lys Thr Ser Trp Glu Lys Gly Ser Leu Val Ser Pro
           835                 840                 845
   Gly Gly Leu Gln Met Leu Leu Val Lys Glu Gly Val Gln Asn Ala Lys
       850                 855                 860
   Thr Asp Val Val Val Asn Ser Val Pro Leu Asp Leu Val Leu Ser Arg
   865                 870                 875                 880
   Gly Pro Leu Ser Lys Ser Leu Leu Glu Lys Ala Gly Pro Glu Leu Gln
                   885                 890                 895
   Glu Glu Leu Asp Thr Val Gly Gln Gly Val Ala Val Ser Met Gly Thr
               900                 905                 910
   Val Leu Lys Thr Ser Ser Trp Asn Leu Asp Cys Arg Tyr Val Leu His
           915                 920                 925
   Val Val Ala Pro Glu Trp Arg Asn Gly Ser Thr Ser Ser Leu Lys Ile
       930                 935                 940
   Met Glu Asp Ile Ile Arg Glu Cys Met Glu Ile Thr Glu Ser Leu Ser
   945                 950                 955                 960
   Leu Lys Ser Ile Ala Phe Pro Ala Ile Gly Thr Gly Asn Leu Gly Phe
                   965                 970                 975
   Pro Lys Asn Ile Phe Ala Glu Leu Ile Ile Ser Glu Val Phe Lys Phe
               980                 985                 990
   Ser Ser Lys Asn Gln Leu Lys Thr  Leu Gln Glu Val His  Phe Leu Leu
           995                 1000                 1005
   His Pro  Ser Asp His Glu Asn  Ile Gln Ala Phe Ser  Asp Glu Phe
       1010                 1015                 1020
   Ala Arg  Arg Ala Asn Gly Asn  Leu Val Ser Asp Lys  Ile Pro Lys
       1025                 1030                 1035
   Ala Lys  Asp Thr Gln Gly Phe  Tyr Gly Thr Val Ser  Ser Pro Asp
       1040                 1045                 1050
   Ser Gly  Val Tyr Glu Met Lys  Ile Gly Ser Ile Ile  Phe Gln Val
       1055                 1060                 1065
   Ala Ser Gly Asp Ile Thr Lys Glu Glu Ala Asp Val Ile Val Asn
   1070 1075 1080
   Ser Thr Ser Asn Ser Phe Asn Leu Lys Ala Gly Val Ser Lys Ala
   1085 1090 1095
   Ile Leu Glu Cys Ala Gly Gln Asn Val Glu Arg Glu Cys Ser Gln
   1100 1105 1110
   Gln Ala Gln Gln Arg Lys Asn Asp Tyr Ile Ile Thr Gly Gly Gly
   1115 1120 1125
   Phe Leu Arg Cys Lys Asn Ile Ile His Val Ile Gly Gly Asn Asp
   1130 1135 1140
   Val Lys Ser Ser Val Ser Ser Val Leu Gln Glu Cys Glu Lys Lys
   1145 1150 1155
   Asn Tyr Ser Ser Ile Cys Leu Pro Ala Ile Gly Thr Gly Asn Ala
   1160 1165 1170
   Lys Gln His Pro Asp Lys Val Ala Glu Ala Ile Ile Asp Ala Ile
   1175 1180 1185
   Glu Asp Phe Val Gln Lys Gly Ser Ala Gln Ser Val Lys Lys Val
   1190 1195 1200
   Lys Val Val Ile Phe Leu Pro Gln Val Leu Asp Val Phe Tyr Ala
   1205 1210 1215
   Asn Met Lys Lys Arg Glu Gly Thr Gln Leu Ser Ser Gln Gln Ser
   1220 1225 1230
   Val Met Ser Lys Leu Ala Ser Phe Leu Gly Phe Ser Lys Gln Ser
   1235 1240 1245
   Pro Gln Lys Lys Asn His Leu Val Leu Glu Lys Lys Thr Glu Ser
   1250 1255 1260
   Ala Thr Phe Arg Val Cys Gly Glu Asn Val Thr Cys Val Glu Tyr
   1265 1270 1275
   Ala Ile Ser Trp Leu Gln Asp Leu Ile Glu Lys Glu Gln Cys Pro
   1280 1285 1290
   Tyr Thr Ser Glu Asp Glu Cys Ile Lys Asp Phe Asp Glu Lys Glu
   1295 1300 1305
   Tyr Gln Glu Leu Asn Glu Leu Gln Lys Lys Leu Asn Ile Asn Ile
   1310 1315 1320
   Ser Leu Asp His Lys Arg Pro Leu Ile Lys Val Leu Gly Ile Ser
       1325                1330                1335
   Arg Asp Val Met Gln Ala Arg Asp Glu Ile Glu Ala Met Ile Lys
       1340                1345                1350
   Arg Val Arg Leu Ala Lys Glu Gln Glu Ser Arg Ala Asp Cys Ile
       1355                1360                1365
   Ser Glu Phe Ile Glu Trp Gln Tyr Asn Asp Asn Asn Thr Ser His
       1370                1375                1380
   Cys Phe Asn Lys Met Thr Asn Leu Lys Leu Glu Asp Ala Arg Arg
       1385                1390                1395
   Glu Lys Lys Lys Thr Val Asp Val Lys Ile Asn His Arg His Tyr
       1400                1405                1410
   Thr Val Asn Leu Asn Thr Tyr Thr Ala Thr Asp Thr Lys Gly His
       1415                1420                1425
   Ser Leu Ser Val Gln Arg Leu Thr Lys Ser Lys Val Asp Ile Pro
       1430                1435                1440
   Ala His Trp Ser Asp Met Lys Gln Gln Asn Phe Cys Val Val Glu
       1445                1450                1455
   Leu Leu Pro Ser Asp Pro Glu Tyr Asn Thr Val Ala Ser Lys Phe
       1460                1465                1470
   Asn Gln Thr Cys Ser His Phe Arg Ile Glu Lys Ile Glu Arg Ile
       1475                1480                1485
   Gln Asn Pro Asp Leu Trp Asn Ser Tyr Gln Ala Lys Lys Lys Thr
       1490                1495                1500
   Met Asp Ala Lys Asn Gly Gln Thr Met Asn Glu Lys Gln Leu Phe
       1505                1510                1515
   His Gly Thr Asp Ala Gly Ser Val Pro His Val Asn Arg Asn Gly
       1520                1525                1530
   Phe Asn Arg Ser Tyr Ala Gly Lys Asn Ala Val Ala Tyr Gly Lys
       1535                1540                1545
   Gly Thr Tyr Phe Ala Val Asn Ala Asn Tyr Ser Ala Asn Asp Thr
       1550                1555                1560
   Tyr Ser Arg Pro Asp Ala Asn Gly Arg Lys His Val Tyr Tyr Val
       1565                1570                1575
   Arg Val Leu Thr Gly Ile Tyr Thr His Gly Asn His Ser Leu Ile
   1580 1585 1590
   Val Pro Pro Ser Lys Asn Pro Gln Asn Pro Thr Asp Leu Tyr Asp
   1595 1600 1605
   Thr Val Thr Asp Asn Val His His Pro Ser Leu Phe Val Ala Phe
   1610 1615 1620
   Tyr Asp Tyr Gln Ala Tyr Pro Glu Tyr Leu Ile Thr Phe Arg Lys
   1625 1630 1635
<210> 4
   <211> 656
   <212> PRT
   <213> Homo sapiens
<400> 4
<210> 5
   <211> 323
   <212> PRT
   <213> Homo sapiens
<400> 5
   Met Gln Pro Ser Gly Trp Ala Ala Ala Arg Glu Ala Ala Gly Arg Asp
   1 5 10 15
   Met Leu Ala Ala Asp Leu Arg Cys Ser Leu Phe Ala Ser Ala Leu Gln
   20 25 30
   Ser Tyr Lys Arg Asp Ser Val Leu Arg Pro Phe Pro Ala Ser Tyr Ala
   35 40 45
   Arg Gly Asp Cys Lys Asp Phe Glu Ala Leu Leu Ala Asp Ala Ser Lys
   50 55 60
   Leu Pro Asn Leu Lys Glu Leu Leu Gln Ser Ser Gly Asp Asn His Lys
   65 70 75 80
   Arg Ala Trp Asp Leu Val Ser Trp Ile Leu Ser Ser Lys Val Leu Thr
   85 90 95
   Ile His Ser Ala Gly Lys Ala Glu Phe Glu Lys Ile Gln Lys Leu Thr
   100 105 110
   Gly Ala Pro His Thr Pro Val Pro Ala Pro Asp Phe Leu Phe Glu Ile
   115 120 125
   Glu Tyr Phe Asp Pro Ala Asn Ala Lys Phe Tyr Glu Thr Lys Gly Glu
   130 135 140
   Arg Asp Leu Ile Tyr Ala Phe His Gly Ser Arg Leu Glu Asn Phe His
   145 150 155 160
   Ser Ile Ile His Asn Gly Leu His Cys His Leu Asn Lys Thr Ser Leu
   165 170 175
   Phe Gly Glu Gly Thr Tyr Leu Thr Ser Asp Leu Ser Leu Ala Leu Ile
   180 185 190
   Tyr Ser Pro His Gly His Gly Trp Gln His Ser Leu Leu Gly Pro Ile
   195 200 205
   Leu Ser Cys Val Ala Val Cys Glu Val Ile Asp His Pro Asp Val Lys
   210 215 220
   Cys Gln Thr Lys Lys Lys Asp Ser Lys Glu Ile Asp Arg Arg Arg Ala
   225 230 235 240
   Arg Ile Lys His Ser Glu Gly Gly Asp Ile Pro Pro Lys Tyr Phe Val
   245 250 255
   Val Thr Asn Asn Gln Leu Leu Arg Val Lys Tyr Leu Leu Val Tyr Ser
   260 265 270
   Gln Lys Pro Pro Lys Ser Arg Ala Ser Ser Gln Leu Ser Trp Phe Ser
   275 280 285
   Ser His Trp Phe Thr Val Met Ile Ser Leu Tyr Leu Leu Leu Leu Leu
   290 295 300
   Ile Val Ser Val Ile Asn Ser Ser Ala Phe Gln His Phe Trp Asn Arg
   305 310 315 320
   Ala Lys Arg

## Description

The present invention relates to the use of mobilization compounds and methods useful for the identification of PARP interacting molecules and for the purification of PARP proteins.

Poly(ADP-ribose) polymerases (PARPs) constitute a family of cell signalling enzymes present in eukaryotes which catalyse poly(ADP-ribosylation) of DNA-binding proteins. PARPS are also known as poly(ADP-ribose) synthethases or poly(ADP-ribose) transferases (pARTs). These enzymes play an important role in the immediate cellular response to DNA damage. In response to DNA damage induced by ionizing radiation, oxidative stress and DNA-binding anti-tumour drugs, PARPs add ADP-ribose units to carboxlate groups of aspartic and glutamic residues of target proteins. This poly(ADP-ribosylation) is a posttranslational modification that triggers the inactivation of the acceptor protein through the attachment of a complex branched polymer of ADP-ribose units (Schreiber et al., 2006. Nature Reviews Cell Biology 7, 517-528).

ADP ribosylation is a posttranslational protein modification in which the ADP-ribose moiety is transferred from NAD onto specific amino acid side chains of target proteins (Schreiber et al., 2006. Nature Reviews Cell Biology 7, 517-528).

PARP1 is the first characterized and the best known member of the PARP family. This protein, encoded by the ADPRT gene, is a highly conserved chromatin bound enzyme which binds nicked DNA and mediates protection against DNA damage. In response to extensive DNA damage, PARP1 is overactivated and induces depletion of cellular NAD+ and ATP leading to cell dysfunction or cell death. Overactivation of PARP1 is involved in the pathogenesis of several diseases including stroke, myocardial infarctation, diabetes, neurodegenerative disorders and inflammatory diseases.

Targeting PARPs with small molecule inhibitors has been proposed as a promising approach for the cancer therapy (Haince et al., 2005. Trends Mol Med. 11(10):456-63).

Several PARP inhibitors have been reported in the literature. However, the first generation of PARP inhibitors is non-selective for the various members of the WARP family and there is a need for selective inhibitors. In addition, new methods for the selectivity profiling of PARP inhibitors are needed.

One prerequisite for the identification and characterization of PARP inhibitors is the provision of suitable assays, preferably using physiological forms of the protein target. In the art, several strategies have been proposed to address this issue.

A two-step affinity purification protocol was described for the purification of PARP proteins from calf thymus (D'Amours et al., 1997. Analytical Biochemistry 249, 106-108). The first step consisted of DNA-cellulose chromatography and the second step of 3-Aminobenzamide affinity chromatography. 3-Aminobenzamide is a PARP inhibitor which interacts with the C-terminal catalytic domain of the enzyme.

The evaluation of kinase inhibitor selectivity employing different approaches to identify kinase inhibitor targets by chemical proteomics, including the immobilization of kinase inhibitors on a chromatography resin, was described (Daub et al., 2004. Assay and Drug Development Technologies 2 (2). 215-224).

Conventionally, PARP enzyme activity can be measured using purified or recombinant enzyme in a solution-based assay with a suitable substrate (Dantzer et al., 2006. Methods in Enzymology 409, 493-510). This type of assay can be used to identify PARP inhibitors, but also to assess inhibitor selectivity by testing an inhibitor against all members of the PARP family.

In view of the above, there is a need for providing effective tools and methods for the identification and selectivity profiling of PARP interacting compounds as well as for the purification of PARP proteins.

The present disclosure describes inter alia an immobilization compound of formula (I) 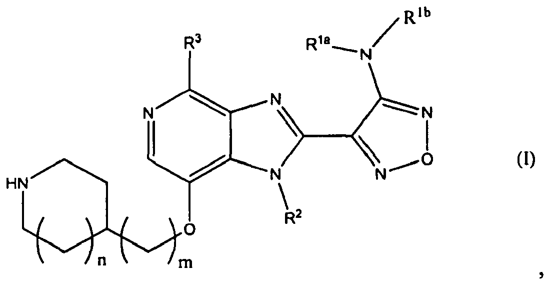 or a salt thereof, wherein
R^{1a}, R^{1b}, R² are independently selected from the group consisting of H; and C₁₋₄ alkyl, wherein C₁₋₄ alkyl is optionally substituted with one or more halogen, which are the same or different;
R³ is H; halogen; CN; C(O)OR⁴; OR⁴ ; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂ OC(O)R⁴; N(R⁴)C(O)R⁴ ; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R⁵, which are the same or different;
R⁴, R^{4a}, R^{4b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R⁵, which are the same or different;
R⁵ is halogen; CN; OR⁶; SR⁶; N(R⁶R^{6a}); or NO₂
R⁶, R^{6a} are independently selected from the group consisting of H; and C₁₋₄ alkyl, wherein C₁₋₄ alkyl is optionally substituted with one or more halogen, whcih are the same or different;
m is 0; 1 ; or 2;
n is 0; 1; or 2.

In case a variable or substituent can be selected from a group of different variants and such variable or substituent occurs more than once the respective variants can be the same or different.

Within the meaning of the present invention the terms are used as follows:

"Alkyl" means a straight-chain or branched saturated hydrocarbon chain. Each hydrogen of an alkyl carbon may be replaced by a substituent.

"Alkenyl" means a straight-chain or branched hydrocarbon chain that contains at least one carbon-carbon double bond. Each hydrogen of an alkenyl carbon may be replaced by a substituent.

"Alkynyl" means a straight-chain or branched hydrocarbon chain, that contains at least one carbon-carbon triple bond. Each hydrogen of an alkynyl carbon may be replaced by a substituent.

"C₁₋₄ alkyl" means an alkyl chain having 1 - 4 carbon atoms, e.g. if present at the end of a molecule: methyl, ethyl, n-propyl, isopropyl, n-butyl, isobutyl, sec-butyl tert-butyl, or e.g. -CH₂-, -CH₂-CH₂-, -CH(CH₃)-, -C(CH₂)-, -CH₂-CH₂-CH₂-, -CH(C₂H₅)-, -CH(CH₃)₂-, when two moieties of a molecule are linked by the alkyl group. Each hydrogen of a C₁₋₄ alkyl carbon may be replaced by a substituent.

"C₁₋₆ alkyl" means an alkyl chain having 1-6 carbon atoms, e.g. if present at the end of a molecule: C₁₋₄ alkyl, methyl, ethyl, n-propyl, isopropyl, n-butyl, isobutyl, sec-butyl, tert-butyl, n-pentyl, n-hexyl, or e.g. -CH₂-, -CH₂-CH₂-, -CH(CH₃)-, -C(CH₂)-, -CH₂-CH₂-CH₂-, -CH(C₂H₅)-, -CH(CH₃)₂-, when two moieties of a molecule are linked by the alkyl group. Each hydrogen of a C₁₋₆ alkyl carbon may be replaced by a substituent.

"C₂₋₆ alkenyl" means an alkenyl chain having 2 to 6 carbon atoms, e.g. if present at the end of a molecule: -CH=CH₂, -CH=CH-CH₃, -CH₂-CH=CH₂, -CH=CH-CH₂-CH₃, -CH=CH-CH=CH₂, or e.g. -CH=CH-, when two moieties of a molecule are linked by the alkenyl group. Each hydrogen of a C₂₋₆ alkenyl carbon may be replaced by a substituent.

"C₂₋₆ alkynyl" means an alkynyl chain having 2 to 6 carbon atoms, e.g. if present at the end of a molecule: -C≡CH, -CH₂-C≡CH, CH₂-CH₂-C≡CH, CH₂-C≡C-CH₃, or e.g. -C≡C- when two moieties of a molecule are linked by the alkynyl group. Each hydrogen of a C₂₋₆ alkynyl carbon may be replaced by a substituent.

"Halogen" means fluoro, chloro, bromo or iodo. It is generally preferred that halogen is fluoro or chloro.

The immobilization compounds described in the present disclosure have been named as "immobilization compounds" due to their preferred use in the preparation of immobilization products as described below. However, other possible uses, e.g. as a soluble competitor in assays or as a labelled probe, are also explicitly included within the present invention.

In case the immobilization compounds according to formula (I) contain one or more acidic or basic groups, the invention also comprises their corresponding salts. Thus, the immobilization compounds of the formula (I) which contain acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Immobilization compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the immobilization compounds of the formula (I) simultaneously contain acidic and basic groups in the molecule, the disclosure also includes, in addition to the salt forms mentioned, inner salts or betaines (zwitterions). The respective salts according to the formula (I) can be obtained by customary methods which are known to the person skilled in the art like, for example by contacting these with an organic or inorganic acid or base in a solvent or dispersant, or by anion exchange or cation exchange with other salts.

The present disclosure furthermore includes all solvates of the immobilization compounds.

As it can be taken from the Examples, immobilization compounds falling under formula (I) have been shown to bind to PARP proteins, which makes them useful tools in the context of assays for the identification of PARP interacting compounds.

Preferred immobilization compounds of formula (I) are those immobilization compounds in which one or more of the residues contained therein have the meanings given below, with all combinations of preferred substituent definitions being a subject of the present invention. With respect to all preferred immobilization compounds of the formulae (I) the present invention also includes all tautomeric and stereoisomeric forms and mixtures thereof in all ratios.

Preferably, the substituents mentioned below independently have the following meaning. Hence, one or more of these substituents can have the preferred or more preferred meanings given below.

Preferably, R^{1a}, R^{1b} are independently selected from the group consisting of H; CH₃; and C₂H₅. In a more preferred embodiment at least one of R^{1a}, R^{1b} is H. In a further more preferred embodiment R^{1a}, R^{1b} are H.

Preferably, R² is H; CH₃; or C₂H₅. In a more preferred embodiment R² is CH₃ or C₂H₅, especially C₂H₅.

Preferably, R³ is halogen, especially chloro; or C₁₋₆ alkynyl, optionally substituted with R⁵.

Preferably R⁵ is OR⁶.

Preferably R⁶ is H; or CH₃.

Even more preferred is R³ chloro; or C≡C-C(CH₃)₂OH.

Preferably, m is 1. Preferably, n is 1. Preferably, n + m is 2.

Preferred immobilization compounds of formula (I) are selected from the group consisting of 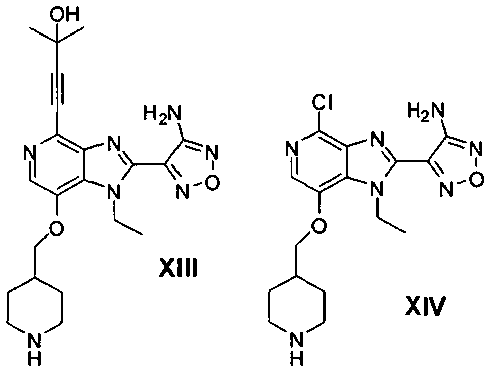 or a mixture of both, preferably in the form of a hydrohalogenide or carboxylate, especially as hydrochloride or formate.

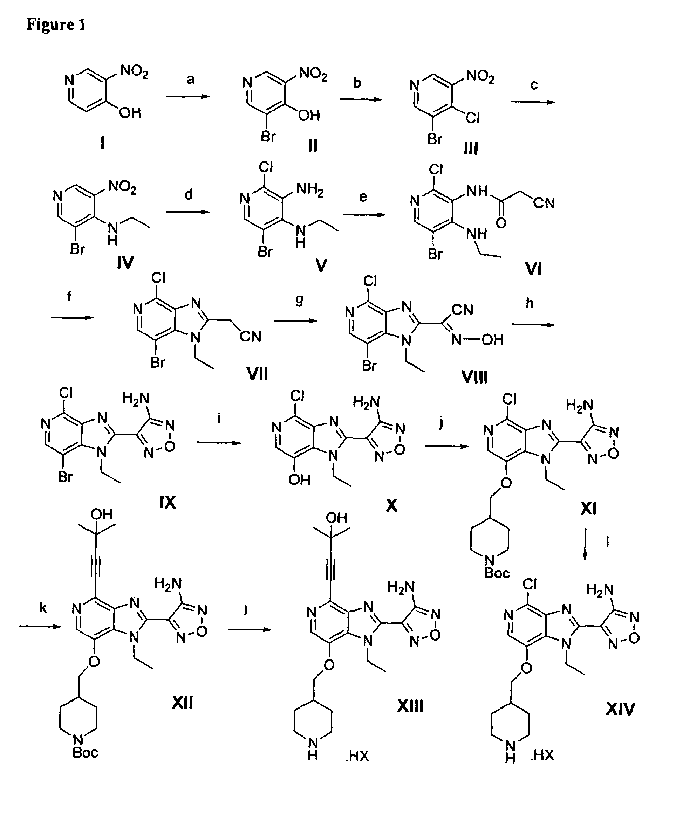

The immobilization compounds can be prepared by methods well known in the art. Exemplary analogous routes for the synthesis are described in Fig. 1.

The disclosure further describes a method for the preparation of an immobilization product, wherein at least one immobilization compound is immobilized on a solid support. Such immobilization products obtainable according to the method are e.g. useful in the methods of the invention for the identification of PARP interacting compounds or in diagnostic methods for the diagnosis of cancer.

According to the method of the disclosure, at least one immobilization compound is immobilized on a solid support. Throughout the invention, the term "solid support" relates to every undissolved support being able to immobilize a small molecule ligand on its surface.

According to the invention, the term "at least one immobilization compound" means either that at least one immobilization compound of the same type is immobilized on the solid support or that one or more different immobilization compounds (each of them either in singular or plural) may be immobilized on the solid support. Preferably, one or two different immobilization compounds are immobilized on the solid support, more preferably the preferred immobilization compounds of formula (I) are selected from the group consisting of

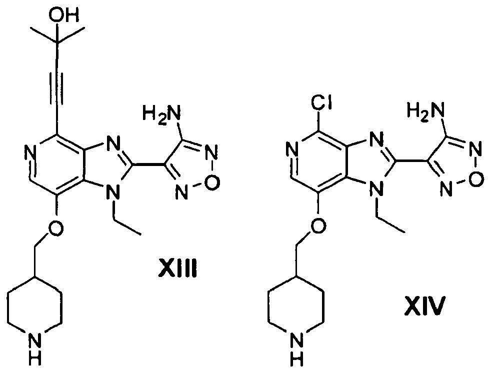

The solid support may be selected from the group consisting of agarose, modified agarose, sepharose beads (e.g. NHS-activated sepharose), latex, cellulose, and ferro- or ferrimagnetic particles.

In case that the solid support is a material comprising various entities, e.g. in case that the solid support comprises several beads or particles, it is envisaged within the present invention that, if different immobilization compounds are immobilized, on each single entity, e.g. each bead or particle, one or more different immobilization compounds are immobilized. Therefore, in case that two immobilization compounds are used, it is envisaged within the present invention that on each single entity one or two different immobilization compounds are immobilized. If no measures are taken that on one entity only one different immobilization compound is immobilized, it is very likely that on each entity all different immobilization compounds will be present.

The immobilization compound or compounds may be coupled to the solid support either covalently or non-covalently. Non-covalent binding includes binding via biotin affinity ligands binding to steptavidin matrices.

Preferably, the immobilization compound or compounds are covalently coupled to the solid support.

Methods for immobilizing compounds on solid supports are known in the art and further exemplified in Example 1.

In general, before the coupling, the matrixes can contain active groups such as NHS, Carbodiimide etc. to enable the coupling reaction with the immobilization compound. The immobilization compound can be coupled to the solid support by direct coupling (e.g. using functional groups such as amino-, sulfhydryl-, carboxyl-, hydroxyl-, aldehyde-, and ketone groups) and by indirect coupling (e.g. via biotin, biotin being covalently attached to the immobilization product and non-covalent binding of biotin to streptavidin which is bound directly to the solid support).

The linkage to the solid support material may involve cleavable and non-cleavable linkers. The cleavage may be achieved by enzymatic cleavage or treatment with suitable chemical methods.

Therefore, the immobilization product results from a covalent direct or linker mediated attachment of the at least one immobilization compound to the solid support.

The linker may be a C₁₋₁₀ alkylene group, which is optionally interrupted by one or more atoms or functional groups selected from the group consisting of S, O, NH, C(O)O, C(O), and C(O)NH and wherein the linker is optionally substituted with one or more substituents independently selected from the group consisting of halogen, OH, NH₂, C(O)H, C(O)NH₂, SO₃H NO₂ and CN.

The term "C₁₋₁₀ alkylene" means an alkylene chain having 1-10 carbon atoms, e.g. methylene, ethylene, -CH=CH-, -C≡C-, n-propylene and the like, wherein each hydrogen of a carbon atom may be replaced by a substituent.

The term "interrupted" means that the one or more atoms or functional groups are inserted between two carbon atoms of the alkylene chain or at the end of said chain.

Preferably, said immobilization occurs via the ring nitrogen atom of the saturated ring in formula (I) above. More preferred, said nitrogen atom is part of an amine functional group, so that the immobilization occurs via amid bond forming of an immobilization compound or a mixture thereof and optionally activated carboxylic acid functional groups of the solid support. Perhaps well known protective group techniques may be required during the immobilization step.

The disclosure further describes an immobilization product, obtainable by the method of the disclosure.

Therefore, an immobilization product which is obtainable by the method of the disclosure is an immobilization compound immobilized on a solid support. This immobilization product will be referred to in the following as the immobilization product and is used in the methods of the present invention.

The immobilization compound or immobilization product may further be labeled.

By "labeled" is meant that the respective substance is either directly or indirectly labeled with a molecule which provides a detection signal, e.g. radioisotope, fluorescent tag, chemiluminescent tag, a peptide or specific binding molecules. Specific binding molecules include pairs, such as biotin and streptavidin, digoxin and antidigoxin. The label can directly or indirectly provide a detectable signal. The tag can also be a peptide which can be used in an enzyme fragment complementation assay (e.g. beta-galactosidase enzyme fragment complementation; Zaman et al., 2006. Assay Drug Dev. Technol. 4(4):411-420). The labeled compounds would be useful not only in imaging techniques but also in assays, both in vitro and in vivo, for identifying PARP interacting compounds by inhibition of binding of the labeled compound, for example in PARP assays that contain such labeled compounds.

Radioisotopes are commonly used in biological applications for the detection of a variety of biomolecules and have proven to be useful in binding assays. Several examples of probes have been designed to incorporate ³H (also written as T for tritium) because it can replace hydrogen in a probe without altering its structure. An "isotopically" or "radiolabeled" compound is a compound of the invention where one or more atoms are replaced or substituted by an atom having an atomic mass or mass number different from the atomic mass or mass number typically found in nature (i.e., naturally occurring). Suitable radionuclides that may be incorporated in compounds of the present invention include but are not limited to ²H (also written D for Deuterium), ¹¹C, ¹³C, ¹⁴C, ¹³N, ¹⁵N, ¹⁵O, ¹⁷O, ¹⁸O, ¹⁸F, ³⁵S, ³⁶Cl, ⁸²Br, ⁷⁵Br, ⁷⁶Br, ⁷⁷Br, ¹²³I, ¹²⁴I, ¹²⁵I and ¹³¹I.
Guidance for the selection and methods for the attachment of fluorescent tags (e.g. fluorescein, rhodamine, dansyl, NBD (nitrobenz-2-oxa-1,3-diazole), BODIPY (dipyrromethene boron difluoride), and cyanine (Cy)-dyes) to small molecule ligands are generally known in the art. The application of fluorescent probes (fluorophores) in assays for high throughput screening (HTS) of enzymes was described (Zaman et al., 2003. Comb. Chem. High Throughput Screen 6(4): 313-320). The change of the fluorescent properties after binding of the fluorescent probe to the target protein can be determined by measuring for example fluorescence polarization, fluorescence resonance energy transfer, or fluorescence lifetime. In addition, the ALPHAScreen technology can be used where the excitation of a donor bead at 680 nm produces singlet oxygen which can diffuse to an acceptor bead undergoing a chemiluminescent reaction (Glickman et al., 2002. J. Biomol. Screen. 7(1):3-10).
As already discussed above, one possible use of the immobilization products is in the context of the identification of PARP. Therefore, the present invention relates to such methods and uses.

In an aspect, the disclosure describes a method for the identification of a PARP interacting compound, comprising the steps of
a) providing a protein preparation containing PARP,
b) contacting the protein preparation with the immobilization product comprising a compound of formula (I) immobilised on a solid support under conditions allowing the formation of a complex between PARP and the immobilization product,
c) incubating the complex with a given compound, and
d) determining whether the compound is able to separate PARP from the immobilization product.

In a first aspect of the methods of the invention, the present invention relates to a method for the identification of a PARP interacting compound, comprising the steps of
a) providing a protein preparation containing PARP,
b) contacting the protein preparation with an immobilization product comprising a compound of formula (I) or a salt thereof as described in the context of the invention, characterized in that the compound of formula (I) is immobilized on a solid support, and with a given compound under conditions allowing the formation of a complex between PARP and the immobilization product, and
c) detecting the complex formed in step b).

In a second aspect of the methods of the invention, the invention provides a method for the identification of a PARP interacting compound, comprising the steps of:
a) providing two aliquots of a protein preparation containing PARP,
b) contacting one aliquot with an immobilization product comprising a compound of formula (I) or a salt thereof as described in the context of the invention, characterized in that the compound of formula (I) is immobilized on a solid support, under conditions allowing the formation of a complex between PARP and the immobilization product,
c) contacting the other aliquot with the immobilization product and with a given compound under conditions allowing the formation of the complex, and
d) determining the amount of the complex formed in steps b) and c).

In a third aspect of the methods of the invention, the invention relates to a method for the identification of a PARP interacting compound, comprising the steps of:
a) providing two aliquots comprising each at least one cell containing PARP,
b) incubating one aliquot with a given compound,
c) harvesting the cells of each aliquot,
d) lysing the cells in order to obtain protein preparations,
e) contacting the protein preparations with an immobilization product comprising a compound of formula (I) or a salt thereof as described in the context of the invention, characterized in that the compound of formula (I) is immobilized on a solid support, under conditions allowing the formation of a complex between PARP and the immobilization product, and
f) determining the amount of the complex formed in each aliquot in step e).

According to the present invention, the term "PARP" denotes one or more, especially all members of the PARP family (e.g. PARP1, PARP2, PARP3, PARP4 (VPARP), PARP5a (tankyrasel), PARP5b (tankyrase 2), PARP5c (tankyrase3), PARP6, PARP7 (TiPARP), PARPB, PARP9 (Bal), PARP 10, PARP 11, PARP 12, PARP 13, PARP 14, PARP 15, PARP16) (Ame et al., 2004. Bioessays 26(8):882-93). However, throughout the invention, it is preferred that PARP means PARP1, PARP10, PARP14, PARP 15 or PARP16, especially the human isoforms thereof. Throughout the invention, the term "isoform" also includes PARP family members.

According to the present invention, the term "PARP" relates to both human and other proteins of this family. The expression especially includes functionally active derivatives thereof, or functionally active fragments thereof, or a homologues thereof, or variants encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1 % SDS for 1.5 hours at 60°C.

The compounds identified in the context of the presence invention may be a ligand for one, some or all isoforms of PARP (see above).

In some aspects of the invention, first a protein preparation containing PARP is provided. The methods of the present invention can be performed with any protein preparation as a starting material, as long as the PARP is solubilized in the preparation. Examples include a liquid mixture of several proteins, a cell lysate, a partial cell lysate which contains not all proteins present in the original cell or a combination of several cell lysates. The term "protein preparation" also includes dissolved purified protein.

The presence of PARP protein species in a protein preparation of interest can be detected on Western blots probed with antibodies that are specifically directed against PARP. In case that PARP is a specific isoform (e.g. PARP1), the presence of said isoform can be determined by an isoform-specific antibody. Such antibodies are known in the art (Cheong et al., 2003. Clin. Cancer. Res. 9(13):5018-27; Ame et al., 1999. J. Biol. Chem. 274(25):1 17860-17868). Alternatively, also mass spectrometry (MS) could be used to detect PARP, especially isoforms of PARP (see below).

Cell lysates or partial cell lysates can be obtained by isolating cell organelles (e.g. nucleus, mitochondria, ribosomes, golgi etc.) first and then preparing protein preparations derived from these organelles. Methods for the isolation of cell organelles are known in the art. In addition, methods for the preparation of cell lysates have been described (Dantzer et al., 2006. Methods in Enzymology 409, 493-510).

In addition, protein preparations can be prepared by fractionation of cell extracts thereby enriching specific types of proteins such as cytoplasmic or membrane proteins.

Furthermore protein preparations from body fluids can be used (e.g. blood, cerebrospinal fluid, peritoneal fluid and urine).

For example whole embryo lysates derived from defined development stages or adult stages of model organisms such as C. elegans can be used. In addition, whole organs such as heart dissected from mice can be the source of protein preparations. These organs can also be perfused in vitro in order to obtain a protein preparation.

Furthermore, the protein preparation may be a preparation containing PARP which has been recombinantely produced. Methods for the production of recombinant proteins in prokaryotic and eukaryotic cells are widely established (Ame et al., 1999. J. Biol. Chem. 274(25):17860-17868).

In a preferred embodiment of the methods of the invention, the provision of a protein preparation includes the steps of harvesting at least one cell containing PARP and lysing the cell.

Suitable cells for this purpose are e.g. those cells or tissues were members of the PARP family are expressed.

Therefore, in a preferred embodiment, cells isolated from peripheral blood represent a suitable biological material. Procedures for the preparation and culture of human lymphocytes and lymphocyte subpopulations obtained from peripheral blood (PBLs) are widely known (W.E Biddison, Chapter 2.2 "Preparation and culture of human lymphocytes" in Current Protocols in Cell Biology, 1998, John Wiley & Sons, Inc.). For example, density gradient centrifugation is a method for the separation of lymphocytes from other blood cell populations (e.g. erythrocytes and granulocytes). Human lymphocyte subpopulations can be isolated via their specific cell surface receptors which can be recognized by monoclonal antibodies. The physical separation method involves coupling of these antibody reagents to magnetic beads which allow the enrichment of cells that are bound by these antibodies (positive selection). The isolated lymphocyte cells can be further cultured and stimulated by adding cytokines to initiate receptor-mediated cell signaling and subsequently phosphorylation of STAT proteins (Schindler et al., 2007. 282(28):20059-20063).

As an alternative to primary human cells cultured cell lines (e.g. MOLT-4 cells, Jurkat or Ramos cells) can be used.

In a preferred embodiment, the cell is part of a cell culture system and methods for the harvest of a cell out of a cell culture system are known in the art (literature supra).

The choice of the cell will mainly depend on the expression of PARP, since it has to be ensured that the protein is principally present in the cell of choice. In order to determine whether a given cell is a suitable starting system for the methods of the invention, methods like Westernblot, PCR-based nucleic acids detection methods, Northernblots and DNA-microarray methods ("DNA chips") might be suitable in order to determine whether a given protein of interest is present in the cell.

The choice of the cell may also be influenced by the purpose of the study. If the in vivo efficacy for a given drug needs to be analyzed then cells or tissues may be selected in which the desired therapeutic effect occurs (e.g. B-cells). By contrast, for the elucidation of protein targets mediating unwanted side effects the cell or tissue may be analysed in which the side effect is observed (e.g. cardiomyocytes, vascular smooth muscle or epithelium cells).

Furthermore, it is envisaged within the present invention that the cell containing PARP may be obtained from an organism, e.g. by biopsy. Corresponding methods are known in the art. For example, a biopsy is a diagnostic procedure used to obtain a small amount of tissue, which can then be examined miscroscopically or with biochemical methods. Biopsies are important to diagnose, classify and stage a disease, but also to evaluate and monitor drug treatment.

It is encompassed within the present invention that by the harvest of the at least one cell, the lysis is performed simultaneously. However, it is equally preferred that the cell is first harvested and then separately lysed.

Methods for the lysis of cells are known in the art. Lysis of different cell types and tissues can be achieved by homogenizers (e.g. Potter-homogenizer), ultrasonic desintegrators, enzymatic lysis, detergents (e.g. NP-40, Triton X-100, CHAPS, SDS), osmotic shock, repeated freezing and thawing, or a combination of these methods.

According to the methods of the invention, the protein preparation containing PARP is contacted with the immobilization product under conditions allowing the formation of a complex between PARP and the immobilization product of the invention.

In the present invention, the term "a complex between PARP and the immobilization product" denotes a complex where the immobilization product interacts with PARP, e.g. by covalent or, most preferred, by non-covalent binding.

The skilled person will know which conditions can be applied in order to enable the formation of said complex.

In the context of the present invention, the term "under conditions allowing the formation of the complex" includes all conditions under which such formation, preferably such binding is possible. This includes the possibility of having the solid support on an immobilized phase and pouring the lysate onto it. In another preferred embodiment, it is also included that the solid support is in a particulate form and mixed with the cell lysate.

In the context of non-covalent binding, the binding between the immobilization product and PARP is, e.g., via salt bridges, hydrogen bonds, hydrophobic interactions or a combination thereof.

In a preferred embodiment, the steps of the formation of said complex are performed under essentially physiological conditions. The physical state of proteins within cells is described in Petty, 1998 (Howard R. Petty, Chapter 1, Unit 1.5 in: Juan S. Bonifacino, Mary Dasso, Joe B. Harford, Jennifer Lippincott-Schwartz, and Kenneth M. Yamada (eds.) Current Protocols in Cell Biology Copyright © 2003 John Wiley & Sons, Inc. All rights reserved. DOI: 10.1002/0471143030.cb0101s00Online Posting Date: May, 2001 Print Publication Date: October, 1998).

The contacting under essentially physiological conditions has the advantage that the interactions between the immobilization product, the cell preparation containing PARP and optionally the compound reflect as much as possible the natural conditions. "Essentially physiological conditions" are *inter alia* those conditions which are present in the original, unprocessed sample material. They include the physiological protein concentration, pH, salt concentration, buffer capacity and post-translational modifications of the proteins involved. The term "essentially physiological conditions" does not require conditions identical to those in the original living organism, wherefrom the sample is derived, but essentially cell-like conditions or conditions close to cellular conditions. The person skilled in the art will, of course, realize that certain constraints may arise due to the experimental set-up which will eventually lead to less cell-like conditions. For example, the eventually necessary disruption of cell walls or cell membranes when taking and processing a sample from a living organism may require conditions which are not identical to the physiological conditions found in the organism. Suitable variations of physiological conditions for practicing the methods of the invention will be apparent to those skilled in the art and are encompassed by the term "essentially physiological conditions" as used herein. In summary, it is to be understood that the term "essentially physiological conditions" relates to conditions close to physiological conditions, as e. g. found in natural cells, but does not necessarily require that these conditions are identical.

For example, "essentially physiological conditions" may comprise 50-200 mM NaCl or KCl, pH 6.5-8.5, 20-37°C, and 0.001-10 mM divalent cation (e.g. Mg++, Ca++,); more preferably about 150 m NaCl or KCl, pH7.2 to 7.6, 5 mM divalent cation and often include 0.01-1.0 percent non-specific protein (e.g. BSA). A non-ionic detergent (Tween, NP-40, Triton-X100) can often be present, usually at about 0.001 to 2%, typically 0.05-0.2% (volume/volume). For general guidance, the following buffered aequous conditions may be applicable: 10-250 mM NaCl, 5-50 mM Tris HCl, pH5-8, with optional addition of divalent cation(s) and/or metal chelators and/or non-ionic detergents.

Preferably, "essentially physiological conditions" mean a pH of from 6.5 to 7.5, preferably from 7.0 to 7.5, and / or a buffer concentration of from 10 to 50 mM, preferably from 25 to 50 mM, and / or a concentration of monovalent salts (e.g. Na or K) of from 120 to 170 mM, preferably 150 mM. Divalent salts (e.g. Mg or Ca) may further be present at a concentration of from 1 to 5 mM, preferably I to 2 mM, wherein more preferably the buffer is selected from the group consisting of Tris-HCl or HEPES.

The skilled person will appreciate that between the individual steps of the methods of the invention, washing steps may be necessary. Such washing is part of the knowledge of the person skilled in the art. The washing serves to remove non-bound components of the cell lysate from the solid support. Nonspecific (e.g. simple ionic) binding interactions can be minimized by adding low levels of detergent or by moderate adjustments to salt concentrations in the wash buffer.

According to the identification methods of the invention, the read-out system is either the detection or determination of PARP (an aspect of the disclosure), the detection of the complex between PARP and the immobilization product (first aspect of the invention), or the determination of the amount of the complex between PARP and the immobilization product (first, second and third aspect of the invention).

In the method according to an aspect of the disclosure, the detection or determination of the amount of separated WARP is preferably indicative for the fact that the compound is able to separate PARP from the immobilization product. This capacity indicates that the respective compound interacts, preferably binds to PARP, which is indicative for its therapeutic potential.

In one embodiment of the method according to the first aspect of the invention, the complex formed during the method of the invention is detected. The fact that such complex is formed preferably indicates that the compound does not completely inhibit the formation of the complex. On the other hand, if no complex is formed, the compound is presumably a strong interactor with PARP, which is indicative for its therapeutic potential.

According to the methods of the first, second and third aspect of the invention the amount of the complex formed during the method is determined. In general, the less complex in the presence of the respective compound is formed, the stronger the respective compound interacts with PARP, which is indicative for its therapeutic potential.

The detection of the complex formed according to the first aspect of the invention can be performed by using labeled antibodies directed against PARP and a suitable readout system.

According to a preferred embodiment of the first aspect of the invention, the complex between PARP and the immobilization product is detected by determining its amount.

In the course of the first, second and third aspect of the invention, it is preferred that PARP is separated from the immobilization product in order to determine the amount of said complex.

According to invention, separating means every action which destroys the interactions between the immobilization compound and PARP. This includes in a preferred embodiment the elution of PARP from the immobilization compound.

The elution can be achieved by using non-specific reagents as described in detail below (ionic strength, pH value, detergents). In addition, it can be tested whether a compound of interest can specifically elute the PARP from the immobilization compound. Such PARP interacting compounds are described further in the following sections.

Such non-specific methods for destroying the interaction are principally known in the art and depend on the nature of the ligand enzyme interaction. Principally, change of ionic strength, the pH value, the temperature or incubation with detergents are suitable methods to dissociate the target enzymes from the immobilized ligand. The application of an elution buffer can dissociate binding partners by extremes of pH value (high or low pH; e.g. lowering pH by using 0.1 M citrate, pH2-3), change of ionic strength (e.g. high salt concentration using NaI, KI, MgCl₂, or KCI), polarity reducing agents which disrupt hydrophobic interactions (e.g. dioxane or ethylene glycol), or denaturing agents (chaotropic salts or detergents such as Sodium-docedyl-sulfate, SDS).

In some cases, the solid support has preferably to be separated from the released material. The individual methods for this depend on the nature of the solid support and are known in the art. If the support material is contained within a column the released material can be collected as column flowthrough. In case the support material is mixed with the lysate components (so called batch procedure) an additional separation step such as gentle centrifugation may be necessary and the released material is collected as supernatant. Alternatively magnetic beads can be used as solid support so that the beads can be eliminated from the sample by using a magnetic device.

In step d) of the method according to an aspect of the disclosure, it is determined if PARP has been separated from the immobilization product of the invention. This may include the detection of PARP or the determination of the amount PARP.

Consequently, at least in preferred embodiments of all identification methods of the invention, methods for the detection of separated PARP or for the determination of its amount are used. Such methods are known in the art and include physico-chemical methods such as protein sequencing (e.g. Edmann degradation), analysis by mass spectrometry methods or immunodetection methods employing antibodies directed against PARP.

Throughout the invention, if an antibody is used in order to detect PARP or in order to determine its amount (e.g. via ELISA), the skilled person will understand that, if a specific isoform of PARP is to be detected or if the amount of a specific isoform of PARP is to be determined, an isoform-specific antibody may be used. As indicated above, such antibodies are known in the art. Furthermore, the skilled person is aware of methods for producing the same.

For example, PARP2 can be specifically detected with an antibody directed at PARP2 that does not crossreact with other PARP family members (Ame et al., 1999. J. Biol. Chem. 274(25):17860-17868).

Alternatively, several isoforms of PARP can be detected by mass spectrometry methods as illustrated in Example 2.

Preferably, PARP is detected or the amount of PARP is determined by mass spectrometry or immunodetection methods.

The identification of proteins with mass spectrometric analysis (mass spectrometry) is known in the art (Shevchenko et al., 1996. Analytical Chemistry 68: 850-858) and is further illustrated in the example section.

Preferably, the mass spectrometry analysis is performed in a quantitative manner, for example by using iTRAQ technology (isobaric tags for relative and absolute quatification) or cICAT (cleavable isotope-coded affinity tags) (Wu et al., 2006. J. Proteome Res. 5, 651 - 658).

According to a further preferred embodiment of the present invention, the characterization by mass spectrometry (MS) is performed by the identification of proteotypic peptides of PARP. The idea is that PARP is digested with proteases and the resulting peptides are determined by MS. As a result, peptide frequencies for peptides from the same source protein differ by a great degree, the most frequently observed peptides that "typically" contribute to the identification of this protein being termed "proteotypic peptide". Therefore, a proteotypic peptide as used in the present invention is an experimentally well observable peptide that uniquely identifies a specific protein or protein isoform.

According to a preferred embodiment, the characterization is performed by comparing the proteotypic peptides obtained in the course of practicing the methods of the invention with known proteotypic peptides. Since, when using fragments prepared by protease digestion for the identification of a protein in MS, usually the same proteotypic peptides are observed for a given enzyme, it is possible to compare the proteotypic peptides obtained for a given sample with the proteotypic peptides already known for enzymes of a given class of enzymes and thereby identifying the enzyme being present in the sample.

As an alternative to mass spectrometry analysis, the eluted PARP (including coeluted binding partners or scaffold proteins), can be detected or its amount can be determined by using a specific antibody directed against PARP (or against an isoform of PARP, see above).

Furthermore, in another preferred embodiment, once the identity of the coeluted binding partner has been established by mass spectrometry analysis, each binding partner can be detected with specific antibodies directed against this protein.

Suitable antibody-based assays include but are not limited to Western blots, ELISA assays, sandwich ELISA assays and antibody arrays or a combination thereof. The establishment of such assays is known in the art (Chapter 11, Immunology, pages 11-1 to 11-30 in: Short Protocols in Molecular Biology. Fourth Edition, Edited by F.M. Ausubel et al., Wiley, New York, 1999).

These assays can not only be configured in a way to detect and quantify a PARP interacting protein of interest (e.g. a catalytic or regulatory subunit of a PARP complex), but also to analyse posttranslational modification patterns such as phosphorylation, ubiquitin modification or poly(ADP)-ribosylation (Affar et al., 1999. Biochimica et Biophysica Acta 1428, 137-146).

Furthermore, the identification methods of the invention involve the use of compounds which are tested for their ability to be a PARP interacting compound.

Principally, according to the present invention, such a compound can be every molecule which is able to interact with PARP, eg. by inhibiting its binding to the immobilization product of the invention. Preferably, the compound has an effect on PARP, e.g. a stimulatory or inhibitory effect.

Preferably, said compound is selected from the group consisting of synthetic or naturally occurring chemical compounds or organic synthetic drugs, more preferably small molecule organic drugs or natural small molecule compounds. Preferably, said compound is identified starting from a library containing such compounds. Then, in the course of the present invention, such a library is screened.

Such small molecules are preferably not proteins or nucleic acids. Preferably, small molecules exhibit a molecular weight of less than 1000 Da, more preferred less than 750 Da, most preferred less than 500 Da.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are known in the art, wherein natural products are used that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fullfill the specific requirements of the individual binding pocket within a domain family it may be necessary to optimise the natural product structure by chemical variation. Solid-phase chemistry is said to become an efficient tool for this optimisation process. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures.

In a preferred embodiment of the first and second aspect of the invention, the PARP containing protein preparation is first incubated with the compound and then with the immobilization product. However, the simultaneous incubation of the compound and the immobilization product of the invention (coincubation) with the PARP containing protein preparation is equally preferred (competitive binding assay).

In case that the incubation with the compound is first, the PARP is preferably first incubated with the compound for 10 to 60 minutes, more preferred 30 to 45 minutes at a temperature of 4°C to 37°C, more preferred 4°C to 25°C, most preferred 4°C. Preferably compounds are used at concentrations ranging from 1 nM to 100 µM, preferably from 10 nM to 10 µM. The second step, contacting with the immobilized ligand, is preferably performed for 10 to 60 minutes at 4°C.

In case of simultaneous incubation, the PARP is preferably simultaneously incubated with the compound and the immobilization product of the invention for 30 to 120 minutes, more preferred 60 to 120 minutes at a temperature of 4°C to 37°C, more preferred 4°C to 25°C, most preferred 4°C. Preferably compounds are used at concentrations ranging from 1 nM to 100 µM, preferably from 10 nM to 10 µM.

Furthermore, steps a) to c) of the first aspect of the invention may be performed with several protein preparations in order to test different compounds. This embodiment is especially interesting in the context of medium or high throughput screenings (see below).

In a preferred embodiment of the method of the invention according to the second or third aspect, the amount of the complex formed in step c) is compared to the amount formed in step b)

In a preferred embodiment of the method of the invention according to the second or third aspect, a reduced amount of the complex formed in step c) in comparison to step b) indicates that PARP is a target of the compound. This results from the fact that in step c) of this method of the invention, the compound competes with the ligand for the binding of PARP. If less PARP is present in the aliquot incubated with the compound, this means preferably that the compound has competed with the inhibitor for the interaction with the enzyme and is, therefore, a direct target of the protein and vice versa.

Preferably, the identification methods of the invention are performed as a medium or high throughput screening.

The interaction compound identified according to the present invention may be further characterized by determining whether it has an effect on PARP, for example on its enzymatic activity (Dantzer et al., 2006. Methods in Enzymology 409, 493-510).

Briefly, PARP enzyme activity (auto poly-ADP-ribosylation) can e.g. be measured by incubating purified PARP in a suitable buffer together with DNaseI treated DNA and [³²P]-NAD at room temperature. The reaction is stopped by adding trichloro acetic acid (TCA) and then filtered through a glass microfibre filter whereby the reaction product polyADP-ribose is retained on the filter. The filter is washed, dried and the amount of radioactivity is measured with a liquid scintillatin counter (Dantzer et al., 2006. Methods in Enzymology 409, 493-510).

The compounds identified according to the present invention may further be optimized (lead optimisation). This subsequent optimisation of such compounds is often accelerated because of the structure-activity relationship (SAR) information encoded in these lead generation libraries. Lead optimisation is often facilitated due to the ready applicability of high-throughput chemistry (HTC) methods for follow-up synthesis.

The disclosure further describes a method for the preparation of a pharmaceutical composition comprising the steps of
a) identifying a PARP interacting compound as described above, and
b) formulating the interacting compound to a pharmaceutical composition.

Therefore, the disclosure describes a method for the preparation of pharmaceutical compositions, which may be administered to a subject in an effective amount. In a preferred aspect, the therapeutic is substantially purified. The subject to be treated is preferably an animal including, but not limited to animals such as cows, pigs, horses, chickens, cats, dogs, etc., and is preferably a mammal, and most preferably human. In a specific embodiment, a non-human mammal is the subject.

The compounds identified according to the invention are useful for the prevention or treatment of diseases where PARP plays a role (for example PARP inhibitors for cancer). Consequently, the present disclosure also describes the use of a compound identified by the methods of the invention for the preparation of a medicament for the treatment of one or more of the above mentioned diseases. Furthermore, the present disclosure describes a pharmaceutical composition comprising said compound.

In general, the pharmaceutical compositions comprise a therapeutically effective amount of a therapeutic, and a pharmaceutically acceptable carrier. The term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

The composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc..

The amount of the therapeutic which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems. In general, suppositories may contain active ingredient in the range of 0.5% to 10% by weight; oral formulations preferably contain 10% to 95% active ingredient.

Various delivery systems are known and can be used to administer a therapeutic of the invention, e.g., encapsulation in liposomes, microparticles, and microcapsules: use of recombinant cells capable of expressing the therapeutic, use of receptor-mediated endocytosis; construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

It may be desirable to administer the pharmaceutical compositions locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. Administration can be by direct injection at the site (or former site) of a malignant tumor or neoplastic or pre-neoplastic tissue.

The invention further relates to a method for the purification of PARP, comprising the steps of
a) providing a protein preparation containing PARP,
b) contacting the protein preparation with an immobilization product comprising a compound of formula (I) or as salt thereof as described in the context of the invention, characterized in that the compound of formula (I) is immobilized on a solid support, under conditions allowing the formation of a complex between PARP and the immobilization product, and
c) separating PARP from the immobilization product.

As mentioned above, it has been surprisingly found that the compound of the invention and therefore also the immobilization product of the invention is a ligand which recognizes PARP proteins. This enables efficient purification methods for PARP proteins.

With respect to PARP, the protein preparation containing PARP, the conditions for contacting with the immobilization product of the invention, the immobilization product of the invention, the complex between PARP and the immobilization product of the invention, the separation of PARP from the immobilization product of the invention, and the detection of PARP or the determination of its amount, the embodiments as defined above for the identification methods of the invention also apply to the purification method of the invention.

In a preferred embodiment, the method of purification further comprises the step of purifying a specific isoform of PARP.

Preferably, said purification is performed using an isoform specific antibody as explained above.

In a preferred embodiment, the purification method of the invention further comprises after step c) the identification of proteins being capable of binding to PARP. This is especially interesting when the formation of a protein complex is performed under essentially physiological conditions, because it is then possible to preserve the natural condition of the enzyme which includes the existence of binding partners, enzyme subunits or post-translational modifications, which can then be identified with the help of mass spectrometry (MS).

Consequently, in a preferred embodiment, the purification method of the invention further comprises after step c) the determination whether the PARP is further posttranslationally modified, e.g. by ubiquitin modification or by poly(ADP)-ribosylation.

The binding proteins or the posttranslational modifications can be determined as explained above for the detection of WARP or the determination of the amount of PARP. Preferably, said methods include mass spectrometry of imunodetection methods as described above.

The invention further relates to a method for determining the presence of PARP in a sample, comprising the steps of:
a) providing a protein preparation expected to contain PARP,
b) contacting the protein preparation with an immobilization product comprising a compound of formula (I) or a salt thereof as described in the context of the invention, characterized in that the compound of formula (I) is immobilized on a solid support, under conditions allowing the formation of a complex between PARP and the immobilization product, and
c) detecting whether PARP has formed a complex with the immobilization product.

In a preferred embodiment of the invention, said detecting in step c) is performed by separating PARP from the immobilization product and further identification of PARP.

Said identification may be performed by mass spectrometry or immunodetection methods as described above.

With respect to PARP, the protein preparation containing PARP, the conditions for contacting with the immobilization product of the invention, the immobilization product of the invention, the complex between PARP and the immobilization product of the invention, the separation of PARP from the immobilization product of the invention, and the detection of PARP or the determination of its amount, the embodiments as defined above for the identification methods of the invention also apply to the purification method of the invention.

The invention further relates to the use of an immobilization product comprising, a compound of formula (I) or a salt thereof as described in the context of the invention, characterized in that the compound of formula (I) is immobilized on a solid support for the identification of PARP interacting compounds and for the purification of PARP proteins. The embodiments as defined above also apply to the uses of the invention.

The disclosure further describes a kit comprising the compound or the immobilization product. Such a kit is especially useful for performing the methods of the invention. Further components of the kit may be antibodies for the detection of PARP proteins, for example antibodies specific for PARP and antibodies directed at phosphorylation sites of PARP proteins. Such antibodies and their use are known in the art and they are commercially available (Cheong et al., 2003. Clin. Cancer. Res. 9(13):5018-27; Ame et al., 1999. J. Biol. Chem. 274(25):17860-17868). In addition, the kit may contain antibodies directed at poly-ADP-ribose (Affar et al., 1999. Biochimica et Biophysica Acta 1428, 137-146; Kawamitsu et al., 1984. Biochemistry 23(16):3771-3777). Furthermore, the kit may contain further auxiliary components like buffers, means for the detection of antibodies, positive controls, etc.. Such components are known in the art.

The invention is further illustrated by the following figures and examples, which are not considered as being limiting for the scope of protection conferred by the claims of the present application. In case where in the following examples the term "affinity matrix" is used, this term refers to an immobilization product as defined in the present patent.

### Short description of the figures

**Figure 1****:** Reaction scheme for the synthesis of 4-(2-(4-amino-1,2,5-oxadiazol-3-yl)-1-ethyl-7-(piperidin-4-ylmethoxy)-1H-imidazo[4,5-c]pyridin-4-yl)-2-methylbut-3-yn-2-ol (XIII) and 4-(4-chloro-1-ethyl-7-(piperidin-4-ylmethoxy)-1H-imidazo[4,5-c]pyridin-2-yl)-1,2,5-oxadiazol-3-amine (XIV). The compounds were synthesized as described in example 1. Step a) Br₂, H₂O, rt then 50°C b) POCl₃, NN-diethylaniline, 0°C then reflux c) Ethylamine, rt d) HCl conc, SnCl₂, reflux e) Cyanoacetic acid, EDC, DCM, N-methylmorpholine, rt f) Acetic acid, 100°C g) NaNO₂, rt h) Hydroxylamine, Et₃N, Dioxane, reflux i) isopropylmagnesium chloride, B(OMe)₃, H₂O₂, -78°C j) tert-butyl 4-(bromomethyl)piperidine-1-carboxylate, CsCO₃, DMF, 40°C k) 2-methyl-3-butyn-2-ol, tetrakis triphenylphosphine palladium (0), Zn, NaI, DBU, DMSO, 80°C I) 4N HCl in Dioxane, MeOH.
**Figure 2****:** Structure of 4-(2-(4-amino-1,2,5-oxadiazol-3-yl)-1-ethyl-7-(piperidin-4-ylmethoxy)-1H-imidazo[4,5-c]pyridin-4-yl)-2-methylbut-3-yn-2-ol (XIII).
**Figure 3****:** Structure of 4-(4-chloro-1-ethyl-7-(piperidin-4-ylmethoxy)-1H-imidazo[4,5-c]pyridin-2-yl)-1,2,5-oxadiazol-3-amine (XIV).
**Figure 4****:** Drug pulldown experiment with the immobilization compound XIII.
   A protein gel after staining with Coomassie brilliant blue is shown. The drug pulldown experiment was performed as described in example 2 with a 1:1 mixture of Jurkat and Ramos cell lysates containing 50 mg of protein. Proteins bound to the affinity matrix were eluted with SDS sample buffer and separated by SDS-polyacrylamide gel electrophoresis. The indicated gel areas were cut out as gel slices, proteins were treated with trypsin and subjected to analysis by mass spectrometry.

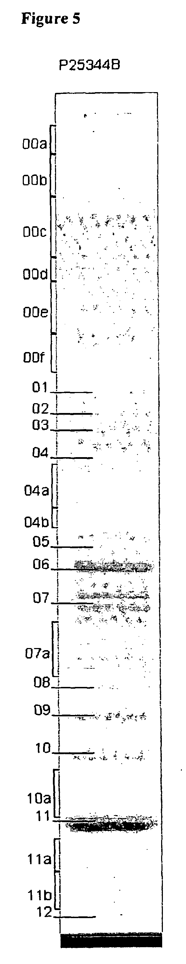

**Figure 5****:** Drug pulldown experiment with the immobilization compound XIV.
   A protein gel after staining with Coomassie brilliant blue is shown. The drug pulldown experiment was performed as described in example 2 with a 1:1 mixture of Jurkat and Ramos cell lysates containing 50 mg of protein. Proteins bound to the affinity matrix were eluted with SDS sample buffer and separated by SDS-polyacrylamide gel electrophoresis. The indicated gel areas were cut out as gel slices, proteins were treated with trypsin and subjected to analysis by mass spectrometry.

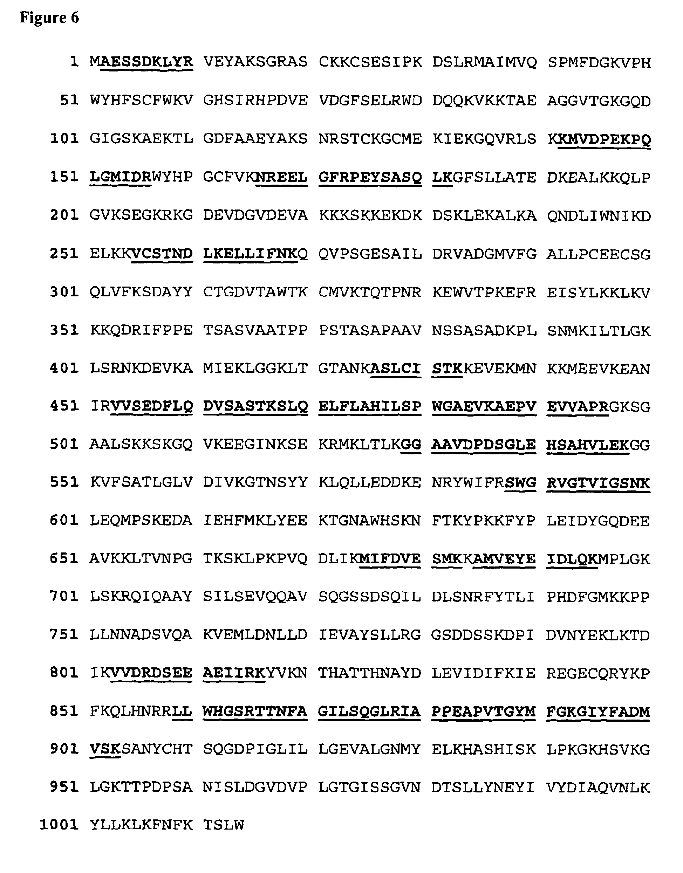

**Figure 6****:** Amino acid sequence of human PARP 1 (IPI00449049.5). Peptides identified by mass spectrometry after a drug pulldown experiment with immobilization compound XIII are shown in bold type and underlined.

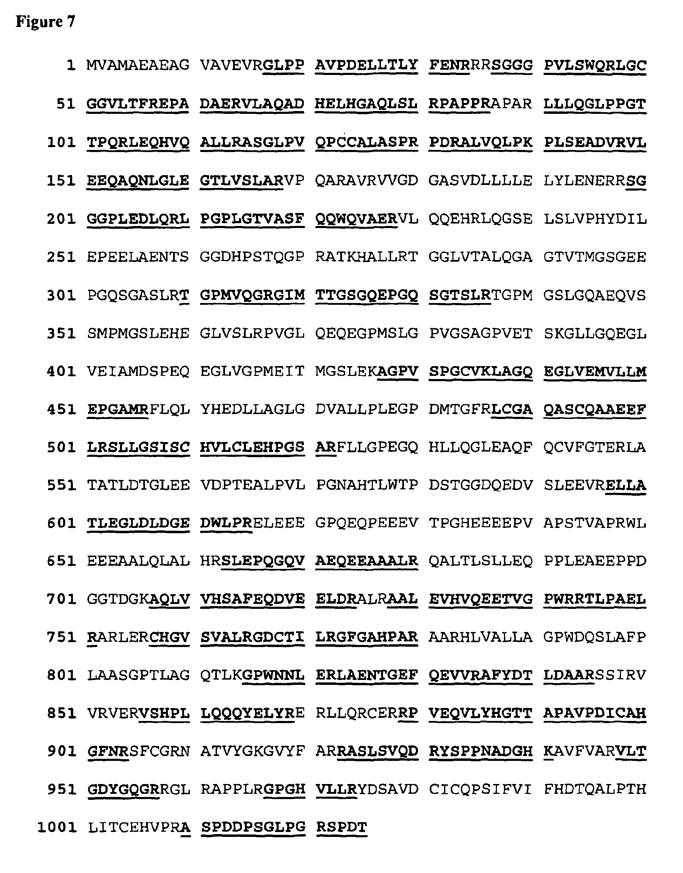

**Figure 7****:** Amino acid sequence of human PARP10 (IP100064457.3). Peptides identified by mass spectrometry after a drug pulldown experiment with immobilization compound XIII are shown in bold type and underlined.

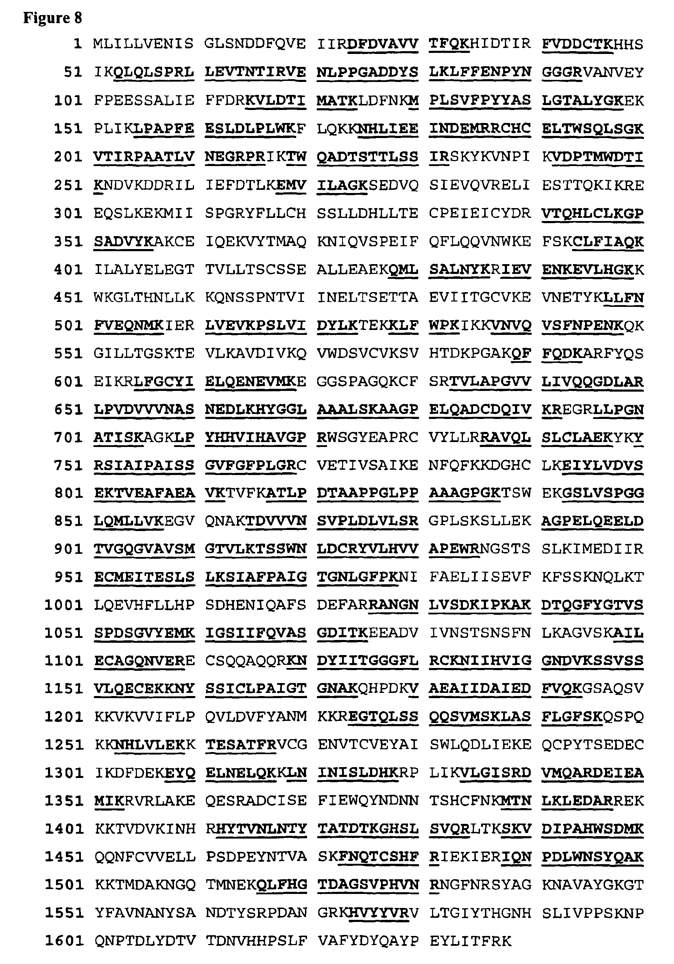

**Figure 8****:** Amino acid sequence of human PARP14 (IPI00291215.5). Peptides identified by mass spectrometry after a drug pulldown with immobilization compound XIII are in bold type and underlined.

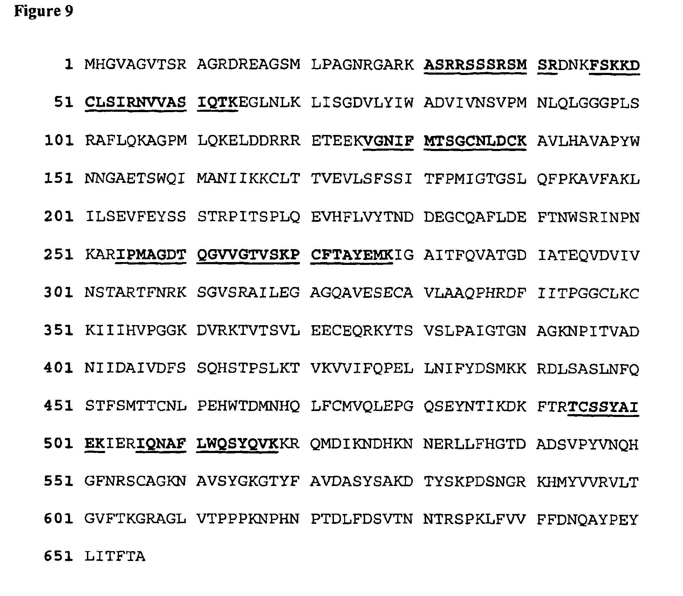

**Figure 9****:** Amino acid sequence of human PARP15 (IPI00166182.3). Peptides identified by mass spectrometry after a drug pulldown with immobilization compound XIII are in bold type and underlined.

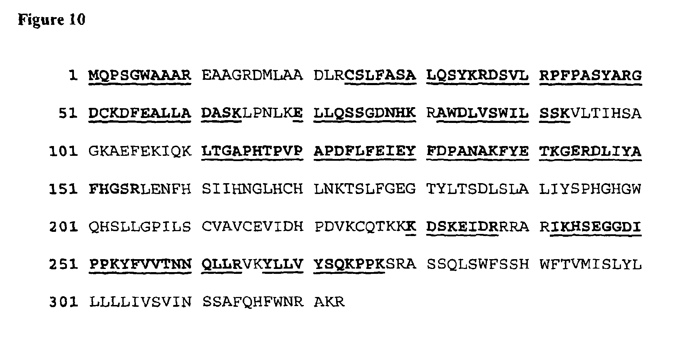

**Figure 10****:** Amino acid sequence of human PARP16 (IPI00297151.3). Peptides identified by mass spectrometry after a drug pulldown with immobilization compound XIII are in bold type and underlined.

### Examples

### Example 1: Preparation of the immobilization compound and immobilization product (affinity matrix)

This example describes the synthesis of compounds and methods for their immobilization on a solid support yielding the immobilization product (affinity matrix) used in the following examples for the capturing of PARP proteins from cell lysates. The synthesis of compounds is illustrated in synthetic scheme 1 (Figure 1).

### Synthesis of 3-bromo-5-nitropyridin-4-ol (II)

To a suspension of 4-hydroxy-3-nitropyridine (I) (7.0g, 50mmol) in water (50ml) was added bromide (3.23ml, 63mmol) drop wise at room temperature. The resulting mixture was stirred for I hour then heated at 50°C for 2 hours. After cooling to room temperature and stirring a further 1 hour, the product was filtered, washed with cold water and dried under vacuum for 3 days to yield the desired product as a white solid (8.35g, 76%). LCMS Rt=0.45mn, no significant MS trace.

### Synthesis of 3-bromo-4-chloro-5-nitropyridine (III)

To phosphorus oxychloride (50ml) cooled in ice was slowly added 3-bromo-5-nitropyridin-4-ol (6.57g, 30mmol). The resulting suspension was stirred at 0°C and N,N-diethylaniline(4.77ml, 30mmol) was added drop wise. The resulting mixture was warmed at room temperature, then refluxed for 2 hours. The resulting black solution was concentrated under vacuum and the residue poured onto ice. The mixture was extracted with ether (200ml). The organic layer was washed with water twice, brine, and dried on MgSO4. After filtration the solvent was removed to yield the desired compound as a brown oil which solidified upon further drying (6.46g, 85%) LCMS Rt=3.18mn, no significant MS trace.

### Synthesis of 3-bromo-N-ethyl-5-nitropyridin-4-amine (IV)

To a solution of 3-bromo-4-chloro-5-nitropyridine (6.45g, 25.4mmol) in THF (19ml) was added slowly a solution of ethylamine in water (70% solution, 101mmol, 13ml). The solution was stirred at room temperature for 3 hours then poured into water. The resulting solution was extracted twice with ethyl acetate. The organic layer was washed with brine, then dried over MgSO4. The solvent was removed. The crude product was purified by flash chromatography (ethyl acetate-Hexane 1:9 to 3:7) to yield the desired compound as a brown oil (5.45g, 92%) LCMS Rt=2.82mn [M+H]⁺=246-248.

### Synthesis of 5-bromo-2-chloro-N4-ethylpyridine-3,4-diamine (V)

3-bromo-N-ethyl-5-nitropyridin-4-amine (4.68g, 19mmol) was dissolved in concentrated hydrochloric acid (47ml) and heated at 85°C. Tin Chloride (10.8g, 57mmol) was added in portions. The reaction was heated at reflux for 1 hour then allowed to cool to room temperature overnight. The off white solid was filtered off then suspended in icy water (90ml). The pH was adjusted to 12 by addition of 12N sodium hydroxide. The resulting solution was extracted with ethyl acetate (2x120ml). The organic layer was washed with brine, and dried over MgSO4. The solvent was removed to yield the desired compound as a yellow oil (3.98g, 83%) LCMS Rt=3.07mn [M+H]⁺=249.9-251.9.

### Synthesis of N-(5-bromo-2-chloro-4-(ethylamino)pyridin-3-yl)-2-cyanoacetamide (VI)

To a solution of 5-bromo-2-chloro-N4-ethylpyridine-3,4- diamine (3.98g, 15.9mmol) in dichloromethane (40ml) was added EDC (4.57g, 23.8mmol), cyanoacetic acid (2.02g, 23.8mmol) and N-methylmorpholine (6.98ml, 63.5mmol). The reaction was stirred at room temperature for 4 hours. The solvent was removed under vacuum. To the slurry was added 150ml of warm ethyl acetate (40°C). The organic layer was washed with 2x 125ml of warm water (40°C), brine (200ml) then dried over MgSO4.. The solvent was partially removed to obtain a slurry which was filtered. The white solid was washed with 2x20ml of cold water and dried in a vacuum oven at 40°C to yield the desired product (3.66g, 72%) LCMS Rt=2.31 mn, [M+H]⁺=316.9-318.9.

### Synthesis of 2-(7-bromo-4-chloro-1-ethyl-1H-imidazo[4,5-c]pyridin-2-yl)acetonitrile (VII)

To N-(5-bromo-2-chloro-4-(ethylamino)pyridin-3-yl)-2-cyanoacetamide (3.15g, 9.9mmol) was added glacial acetic acid (35ml). The suspension was stirred at reflux for 3 hours. The resulting solution was cooled to room temperature to yield the desired product which was used in the next step in situ. LCMS Rt=2.93mn [M+H]⁺= 298.8-300.8.

### Synthesis of 7-bromo-4-chloro-1-ethyl-N-hydroxy-1H-imidazo[4,5-c]pyridine-2-carbimidoyl cyanide (VIII)

To the solution of 2-(7-bromo-4-chloro-1-ethyl-1H-imidazo[4,5-c]pyridin-2-yl)acetonitrile was added slowly NaNO₂ (1.7g, 24.8mmol). The reaction was then stirred at room temperature overnight. The resulting solid was filtered, washed with water, and dried in a vacuum oven at 40°C to yield the desired compound (3.16g, 97% from VI). LCMS Rt=3.58mn [M+H]⁺=327.8-329.8.

### Synthesis of 4-(7-bromo-4-chloro-1-ethyl-1H-imidazo[4,5-c]pyridin-2-yl)-1,2,5-oxadiazol-3-amine (IX)

To a stirred mixture of 7-bromo-4-chloro-1-ethyl-N-hydroxy-1H-imidazo[4,5-c]pyridine-2-carbimidoyl cyanide (3.12g, 9.5mmol) in dioxane (30ml) and triethylamine (5.72ml, 41mmol) was added a solution of hydroxylamine (50% in water, 1.5ml). The reaction was stirred at reflux for 6 hours. The reaction was then cooled to room temperature. The mixture was filtered and the filtrate evaporated to give a yellow solid. The solid was suspended in methanol (12ml), warmed and stirred at 65°C for 30mn, then filtered to yield the desired product as a yellow solid (1.82g, 56%) LCMS Rt=4.15mn [M+H]⁺=343-347.

### Synthesis of 2-(4-amino-1,2,5-oxadiazol-3-yl)-4-chloro-1-ethyl-1H-imidazo[4,5-c]pyridin-7-ol (X)

To a suspension of 4-(7-bromo-4-chloro-1-ethyl-1H-imidazo[4,5-c]pyridin-2-yl)-1,2,5-oxadiazol-3-amine (1.82g,5.3 mmol) in tetrahyfrofuran (46ml) at -78°C was slowly added isopropylmagnesium chloride (2M in THF, 8.5ml, 16.9mmol). The temperature was kept below -70°c during the addition. After stirring 10mn at -78°C, trimethyl borate (2.12ml, 18.5mmol) was added and the reaction then stirred at -78°C for 1 hour. The mixture was then warmed to room temperature and stirred overnight. The reaction was cooled at 0°C. Hydrogen peroxide (50% in water, 5.46ml) and sodium hydroxide 3N (3.64ml) were added together, keeping the temperature of the reaction below 40°C. The resulting mixture was stirred vigorously at room temperature for 2 hours. The organic solvent was removed under vacuum (water left) and the resulting suspension was acidified to pH=1 by addition of 1N hydrochloric acid. The mixture was stirred at room temperature for 30mn. 45ml of ethyl acetate were added and the reaction was stirred at room temperature a further 1 hour. The mixture was filtered (2 crops). The solid was washed with 9ml of water, 9ml of ethyl acetate, 9ml of toluene, and 9ml of ethyl acetate. After drying in a vacuum oven at 40°C, the desired compound was obtained as a yellow solid (1.31m, 89%). LCMS Rt= 3.26mn, [M+H]⁺=281-283.

### Synthesis of tert-butyl 4-((2-(4-amino-1,2,5-oxadiazol-3-yl)-4-chloro-1-ethyl-1H-imidazo[4,5-c]pyridin-7-yloxy)methyl)piperidine-1-carboxylate (XI)

To a solution of 2-(4-amino-1,2,5-oxadiazol-3-yl)-4-chloro-1-ethyl-1H-imidazo[4,5-c]pyridin-7-ol (0.5g, 1.78mmol) in dimethylformamide (15ml) was added cesium carbonate (1.45g, 4.45mmol) followed by tert-butyl 4-(bromomethyl)piperidine-1-carboxylate (0.545 g, 1.96mmol). The reaction was stirred at 40°C for 20 hours. The reaction was then partitioned between ethyl acetate and water. The aqueous layer was extracted twice with ethyl acetate. The combined organic layers were washed with brine and dried over MgSO4. The solvent was removed to give a yellow solid which was triturated with hexane/ethyl acetate to give the desired compound after filtation (0.497g, 58%). LCMS Rt=4.97mn, only fragments available.

### Synthesis of tert-butyl 4-((2-(4-amino-1,2,5-oxadiazol-3-yl)-1-ethyl-4-(3-hydroxy-3-methylbut-1-ynyl)-1H-imidazo[4,5-c]pyridin-7-yloxy)methyl)piperidine-1-carboxylate (XII)

In a microwave tube, tert-butyl 4-((2-(4-amino-1,2,5-oxadiazol-3-yl)-4-chloro-1-ethyl-1H-imidazo[4,5-c]pyridin-7-yloxy)methyl)piperidine-1-carboxylate (47mg, 01mmol), Zinc dust (0.002g, 0.0033mmol), Sodium iodide (0.005g, 0.003mmol), DBU (0.023ml, 0.15mmol), triethylamine (0.017ml, 0.125mmol), 2-methyl-3-butyn-2-ol (0.023ml, 0.24mmol), tetrakis triphenylphosphine palladium (0) (10% weight, 0.005g) and DMSO (0.6ml) were mixed together. The solution was degazed, then the tube was sealed and the reaction heated in a microwave at 80°C for 2.5 hours. The reaction was diluted with ethyl acetate, washed with water. The aqueous layer was extracted twice with ethyl acetate. The combined organic layers were washed with water, brine, then dried over MgSO4. The solvent was removed to give a crude product which was purified by prep HPLC to yield the desired compound (0.012g, 23%) LCMS Rt=4.43mn, only fragments available.

### Synthesis of 4-(2-(4-amino-1,2,5-oxadiazol-3-yl)-1-ethyl-7-(piperidin-4-ylmethoxy)-1H-imidazo[4,5-c]pyridin-4-yl)-2-methylbut-3-yn-2-ol hydrochloride salt (XIII)

tert-butyl 4-((2-(4-amino-1,2,5-oxadiazol-3-yl)-1-ethyl-4-(3-hydroxy-3-methylbut-1-ynyl)-1H-imidazo[4,5-c]pyridin-7-yloxy)methyl)piperidine-1-carboxylate (12mg, 0.023mmol) was dissolved in methanol (3ml). HCl 4N in dioxane (2ml) was added and the reaction was stirred at room temperature 2 hours. The solvent was removed under vacuum. The resulting solid was triturated in methanol (1.5ml), filtered, rinsed with cold methanol (0.5ml) to yield the desired compound as a white solid (9.7mg, 92%). LCMS Rt=2.18mn. Only fragments available. ¹H NMR (d6-DMSO, 400MHz)δ= 8.96(s, br, 2H), 8.23(s, 1H), 7.05(s, br, 2H), 4.83(q, 2H), 4.28(d, 2H), 3.35(m, 2H), 2.96(m, 2H), 2.21(s, br, 1H), 1.97(d, 2H), 1.68(m, 2H), 1.56(s, 6H), 1.47(t, 3H).

### Synthesis of 4-(4-chloro-1-ethyl-7-(piperidin-4-ylmethoxy)-1H-imidazo[4,5-c]pyridin-2-yl)-1,2,5-oxadiazol-3-amine formate salt (XIV)

To a solution of tert-butyl 4-((2-(4-amino-1,2,5-oxadiazol-3-yl)-4-chloro-1-ethyl-1H-imidazo[4,5-c]pyridin-7-yloxy)methyl)piperidine-1-carboxylate (0.045g, 0.094mmol) in methanol (4ml) was added HCl 4N in dioxane (2ml) and the reaction was stirred at room temperature for 3 hours. The solvent was removed and the residue triturated with methanol (2.5ml). The residue was filtered then purified by prep HPLC to yield the desired product as a white powder (0.0185g, 46%). LCMS Rt=2.28mn. Only fragments available.. ¹H NMR (d6-DMSO, 400MHz)δ= 8.40(s, 1H), 8.01 (s, 1H), 6.96(s, 2H), 4.84(q, 2H), 4.19(d, 2H), 3.24(m, 2H), 2.79(m, 2H), 2.12(s, br, 1H), 1.89(m, 2H), 1.6-1.3(m+t, 5H).

**Table 1: Abbreviations**

| | |
|---|---|
| Br₂ | Bromine |
| H₂O | Water |
| POCl₃ | Phosphorus oxychloride |
| HCl | Hydrochloric acid |
| SnCl₂ | Tin chloride |
| EDC | 1-ethyl-3-(3-dimethylaminopropyl) carbodiimide hydrochloride |
| DCM | Dichloromethane |
| NaNO₂ | Sodium nitrite |
| Et₃N | Triethylamine |
| B(OMe)₃ | Trimethoxyborate |
| H₂O₂ | Hydrogen peroxide |
| CsCO₃ | Cesium carbonate |
| DMF | Dimethyl formamide |
| THF | Tetrahydrofuran |
| Zn | Zinc |
| NaI | Sodium iodide |
| DBU | 1,8-Diazabicyclo[5.4.0]undec-7-ene |
| DMSO | Dimethyl sulfoxide |
| MeOH | Methanol |
| MgSO₄ | Magnesium sulfate |
| LCMS | Liquid chromatography Mass Spectroscopy |
| Rt | Retention time |
| NMR | Nuclear Magnetic resonance |
| MHz | Megahertz |
| s | Singlet |
| d | Doublet |
| t | Triplet |
| q | Quadruplet |
| m | Multiplet |
| H | Hydrogen |
| br | Broad |
| MW | Molecular weight |
| V | Volt |
| amu | Atomic mass unit |
| ml | Millilitre |
| min | Minute |
| mmol | Millimole |
| g | Gram |
| API | Atomic pressure ionisation |
| ES | Electrospray |

### NMR, LCMS and prep HPLC conditions:

- All reactions were carried out under inert atmosphere.
- NMR spectra were obtained on a Bruker dpx400.
- The LCMS analytical samples were run on an Agilent HP 1100 - LC/MSD VL system using the following conditions:
   Column: Phenomenex Gemini C18 30 x 3mm 3µm
   Solvents: A= Water + 0.1% Formic acid; B= Acetonitrile + 0.1% Formic acid
   Flow Rate: 1.2ml/min
   Temperature: 40°C

**Table 2: Gradient conditions**

| Time (min) | %A | %B |
|---|---|---|
| 0.00 | 95.0 | 5.0 |
| 6.00 | 5.0 | 95.0 |
| 7.50 | 5.0 | 95.0 |
| 7.60 | 95.0 | 5.0 |
| 8.00 | 95.0 | 5.0 |

### Wavelength:

254nm (reference at 400nm)
210nm (reference at 360nm)

### Mass spectrometry conditions:

The mass spec data were gathered in positive mode, scanning for masses between 150 and 700amu, using a fragmentor ramp from 118V to 400V (for MW= 118.09 to MW= 922.01 respectively). The prepared samples were run on a Waters - ZQ prep system using the following conditions:
Column: Phenomenex Gemini C18 100 x 21.20mm 5µm
Solvents: A= Water + 0.1% Formic acid; B= (95% Acetonitrile: 5% Water) + 0.1% Formic acid
Flow Rate: 20ml/min
Temperature: Room temperature
Gradient conditions: The gradient conditions were variable depending on the retention time of each compound
Wavelength: PDA detection from 190-600nm
Mass spec conditions:
   The mass spec data were gathered in positive and negative mode, from 150 to 700 amu, using API and ES modes.

### Immobilization on beads (immobilization product; affinity matrix)

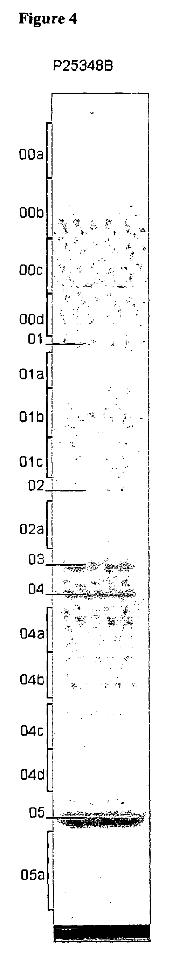

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads was placed in a 15 ml Falcon tube, compound stock solution (mixture of two isomers as shown in Figure 4; usually 100 mM in DMF or DMSO) was added (final concentration 0.2-2 µmol/ml beads) as well as 15 µl of triethylamine (Sigma, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at room temperature on the end-over-end shaker over night. Beads were washed with 10 ml of DMSO and were stored in isopropanol at -20°C. These beads were used as the affinity matrix in example 2 and 3. Control beads (no ligand immobilized) were generated by blocking the NHS-groups by incubation with aminoethanol as described above.

### Example 2: Drug pulldown of PARP proteins using immobilized compounds

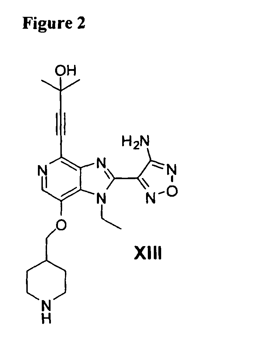

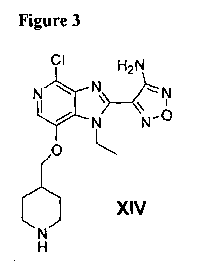

This example demonstrates the use of the immobilized compounds (see Figure 2 and 3) for the capturing and identification of the PARP proteins from mixed Jurkat and Ramos cell lysate. To this end, a mixture of lysates of Jurkat and Ramos cells was contacted with the immobilization product (affinity matrix) described in example 1. Proteins bound to the immobilized compounds were identified by mass spectrometry (MS) analysis. Further experimental protocols can be found in WO2006/134056.

For the identification of proteins by mass spectrometry analysis the proteins captured by the affinity matrix were eluted in SDS sample buffer and subsequently separated by SDS-Polyacrylamide gel elecrophoresis (Figure 4 and 5). Suitable gel bands were cut out and subjected to in-gel proteolytic digestion with trypsin and analyzed by LC-MS/MS mass spectrometry. The identification of PARP protein derived peptides by mass spectrometry is documented in table 4 and the peptide sequence coverage of the PARP protein sequences is shown in Figures 6 to 10.

### 1. Cell culture

Jurkat cells (ATCC number TIB-152) and Ramos cells (ATCC number CRL-1596) were either obtained from an external supplier (CIL SA, Mons, Belgium) or grown in one litre Spinner flasks (Integra Biosciences, #182101) in suspension in RPMI 1640 medium (Invitrogen, #21875-034) supplemented with 10% Fetal Bovine Serum (Invitrogen, #10270-106) at a density between 0.2 x 10⁶ and 1.0 x 10⁶ cells/ml. Cells were harvested by centrifugation, washed once with 1 x PBS buffer (Invitrogen, #14190-094) and cell pellets were frozen in liquid nitrogen and subsequently stored at -80°C.

### 2. Preparation of cell lysates

Cells were homogenized in a Potter S homogenizer in lysis buffer: 50 mM Tris-HCl, 0.8% NP40, 5% glycerol, 150 mM NaCl, 1.5 mM MgCl₂, 25 mM NaF, 1 mM sodium vanadate, 1 mM DTT, pH 7.5. One complete EDTA-free tablet (protease inhibitor cocktail, Roche Diagnostics, 1 873 580) per 25 ml buffer was added. The material was dounced 20 times using a mechanized POTTER S, transferred to 50 ml falcon tubes, incubated for 30 minutes on ice and spun down for 10 minutes at 20,000 x g at 4°C (10,000 rpm in Sorvall SLA600, precooled). The supernatant was transferred to an ultracentrifuge (UZ)-polycarbonate tube (Beckmann, 355654) and spun for 1 hour at 160.000 x g at 4°C (42.000 rpm in Ti50.2, precooled). The supernatant was transferred again to a fresh 50 ml falcon tube, the protein concentration was determined by a Bradford assay (BioRad) and samples containing 50 mg of protein per aliquot were prepared. The samples were immediately used for experiments or frozen in liquid nitrogen and stored frozen at -80°C. This procedure was applied for the preparation of Ramos and Jurkat cell lysates.

### 3. Drug pull-down experiment

Sepharose-beads with the immobilized ligand (100 µl beads per pull-down experiment) were equilibrated in lysis buffer and incubated with a cell lysate sample containing 50 mg of protein on an end-over-end shaker (Roto Shake Genie, Scientific Industries Inc.) for 2 hours at 4°C. Beads were collected, transfered to Mobicol-columns (MoBiTech 10055) and washed with 10 ml lysis buffer containing 0.4% NP40 detergent, followed by 5 ml lysis buffer with 0.2 % detergent. To elute the bound protein, 60 µl 2 x SDS sample buffer was added, the column was heated for 30 minutes at 50°C and the eluate was transferred to a microfuge tube by centrifugation. Proteins were then alkylated with 108 mM iodoacetamid. Proteins were then separated by SDS-Polyacrylamide electrophoresis (SDS-PAGE).

### 4. Protein Identification by Mass Spectrometry

### 4.1 Protein digestion prior to mass spectrometric analysis

Gel-separated proteins were reduced and digested in gel essentially following the procedure described by Shevchenko et al., 1996, Anal. Chem. 68:850-858. Briefly, gel-separated proteins were excised from the gel using a clean scalpel, reduced using 10 mM DTT (in 5mM ammonium bicarbonate, 54°C, 45 minutes) at room temperature in the dark. The reduced proteins were digested in gel with porcine trypsin (Promega) at a protease concentration of 12.5 ng/µl in 5mM ammonium bicarbonate. Digestion was allowed to proceed for 4 hours at 37°C and the reaction was subsequently stopped using 5 µl 5% formic acid.

### 4.2 Sample preparation prior to analysis by mass spectrometry

Gel plugs were extracted twice with 20 µl 1% TFA and pooled with acidified digest supernatants. Samples were dried in a a vaccuum centrifuge and resuspended in 10 µl 0.1 % formic acid.

### 4.3. Mass spectrometric data acquisition

Peptide samples were injected into a nano LC system (CapLC, Waters or Ultimate, Dionex) which was directly coupled either to a quadrupole TOF (QTOF2, QTOF Ultima, QTOF Micro, Micromass) or ion trap (LCQ Deca XP) mass spectrometer. Peptides were separated on the LC system using a gradient of aqueous and organic solvents (see below). Solvent A was 5% acetonitrile in 0.5% formic acid and solvent B was 70% acetonitrile in 0.5% formic acid.

**Table 3: Peptides eluting off the LC system were partially sequenced within the mass spectrometer**

| Time (min) | % solvent A | % solvent B |
|---|---|---|
| 0 | 95 | 5 |
| 5.33 | 92 | 8 |
| 35 | 50 | 50 |
| 36 | 20 | 80 |
| 40 | 20 | 80 |
| 41 | 95 | 5 |
| 50 | 95 | 5 |

### 4.4. Protein identification

The peptide mass and fragmentation data generated in the LC-MS/MS experiments were used to query fasta formatted protein and nucleotide sequence databases maintained and updated regularly at the NCBI (for the NCBInr, dbEST and the human and mouse genomes) and European Bioinformatics Institute (EBI, for the human, mouse, D. melanogaster and C. elegans proteome databases). Proteins were identified by correlating the measured peptide mass and fragmentation data with the same data computed from the entries in the database using the software tool Mascot (Matrix Science; Perkins et al., 1999. Probability-based protein identification by searching sequence databases using mass spectrometry data. Electrophoresis 20, 3551-3567). Search criteria varied depending on which mass spectrometer was used for the analysis. Sequence identifiers are defined by the International Protein Index (IPI) (Kersey et al., 2004. Proteomics 4(7): 1985-1988).

**Table 4: Identification of PARP proteins by mass spectrometry in drug pulldown experiments with immobilized compounds XIII and XIV**

| PARP protein | Compound XIII | Compound XIV |
|---|---|---|
| PARP1 | + | + |
| PARP10 | + | + |
| PARP14 | + | + |
| PARP15 | + | - |
| PARP16 | + | + |

## Claims

1. A method for the identification of a PARP interacting compound, comprising the steps of
a) providing a protein preparation containing PARP,
b) contacting the protein preparation with an immobilization product comprising a compound of formula (I) 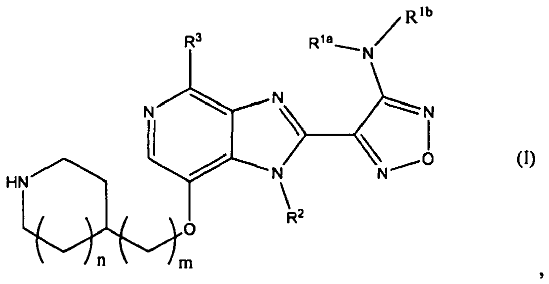 or a salt thereof, wherein
R^{1a}, R^{1b}, R² are independently selected from the group consisting of H; and C₁₋₄ alkyl, wherein C₁₋₄ alkyl is optionally substituted with one or more halogen, which are the same or different;
R³ is H; halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R⁵, which are the same or different;
R⁴, R^{4a}, R^{4b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R⁵, which are the same or different;
R⁵ is halogen; CN; OR⁶; SR⁶; N(R⁶R^{6a}); or NO₂;
R⁶, R^{6a} are independently selected from the group consisting of H; and C₁₋₄ alkyl, wherein C₁₋₄ alkyl is optionally substituted with one or more halogen, which are the same or different;
m is 0; 1; or 2;
n is 0; 1; or 2;
**characterized in that** the compound of formula (I) is immobilized on a solid support,
and with a given compound under conditions allowing the formation of a complex between PARP and the immobilization product, and
c) detecting the complex formed in step b).

2. A method for the identification of a PARP interacting compound, comprising the steps of:
a) providing two aliquots of a protein preparation containing PARP,
b) contacting one aliquot with an immobilization product comprising a compound of formula (I) 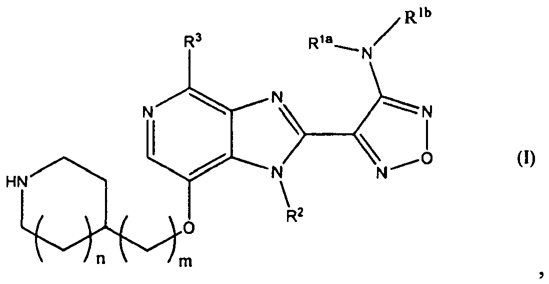 or a salt thereof, wherein
R^{1a}, R^{1b}, R² are independently selected from the group consisting of H; and C₁₋₄ alkyl, wherein C₁₋₄ alkyl is optionally substituted with one or more halogen, which are the same or different;
R³ is H; halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R⁵, which are the same or different;
R⁴, R^{4a}, R^{4b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R⁵, which are the same or different;
R⁵ is halogen; CN; OR⁶; SR⁶; N(R⁶R^{6a}); or NO₂;
R⁶, R^{6a} are independently selected from the group consisting of H; and C₁₋₄ alkyl, wherein C₁₋₄ alkyl is optionally substituted with one or more halogen, which are the same or different;
m is 0; 1; or 2;
n is 0; 1; or 2;
**characterized in that** the compound of formula (I) is immobilized on a solid support,
under conditions allowing the formation of a complex between PARP and the immobilization product,
c) contacting the other aliquot with the immobilization product and with a given compound under conditions allowing the formation of the complex, and
d) determining the amount of the complex formed in steps b) and c).

3. A method for the identification of a PARP interacting compound, comprising the steps of:
a) providing two aliquots comprising each at least one cell containing PARP,
b) incubating one aliquot with a given compound,
c) harvesting the cells of each aliquot,
d) lysing the cells in order to obtain protein preparations,
e) contacting the protein preparations with an immobilization product comprising a compound of formula (I) 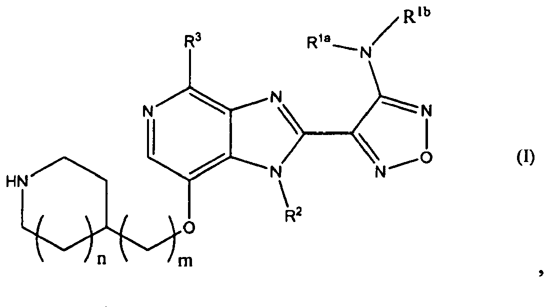 or a salt thereof, wherein
R^{1a}, R^{1b}, R² are independently selected from the group consisting of H; and C₁₋₄ alkyl, wherein C₁₋₄ alkyl is optionally substituted with one or more halogen, which are the same or different;
R³ is H; halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R⁵, which are the same or different;
R^{a}, R^{4a}, R^{4b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R⁵, which are the same or different;
R⁵ is halogen; CN; OR⁶; SR⁶; N(R⁶R^{6a}); or NO₂;
R⁶, R^{6a} are independently selected from the group consisting of H; and C₁₋₄ alkyl, wherein C₁₋₄ alkyl is optionally substituted with one or more halogen, which are the same or different;
m is 0; 1; or 2;
n is 0; 1; or 2;
**characterized in that** the compound of formula (I) is immobilized on a solid support,
under conditions allowing the formation of a complex between PARP and the immobilization product, and
f) determining the amount of the complex formed in each aliquot in step e).

4. The method of any of claims 2 or 3, wherein a reduced amount of the complex formed in the aliquot incubated with the compound in comparison to the aliquot not incubated with the compound indicates that PARP is a target of the compound.

5. The method of any of claims 2 to 4, wherein the amount of the complex is determined by separating PARP from the immobilization product and subsequent detection of separated PARP or subsequent determination of the amount of separated PARP, in particular wherein PARP is detected or the amount of PARP is determined by mass spectrometry or immunodetection methods, preferably with an antibody directed against PARP.

6. The method of any of claims 2 to 5, wherein said given compound is selected from the group consisting of synthetic compounds, or organic synthetic drugs, more preferably small molecule organic drugs, and natural small molecule compounds.

7. The method of any of claims 2 to 6, wherein the given compound is a PARP inhibitor.

8. A method for the purification of PARP, comprising the steps of
a) providing a protein preparation containing PARP,
b) contacting the protein preparation with an immobilization product comprising a compound of formula (I) 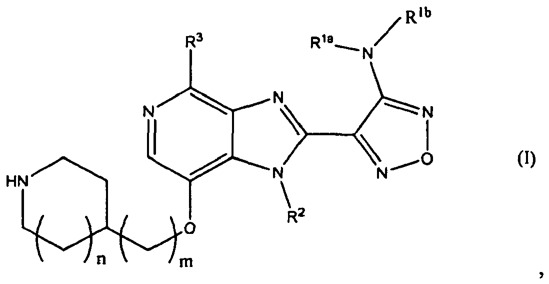 or a salt thereof, wherein
R^{1a}, R^{1b}, R² are independently selected from the group consisting of H; and C₁₋₄ alkyl, wherein C₁₋₄ alkyl is optionally substituted with one or more halogen, which are the same or different;
R³ is H; halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R⁵, which are the same or different;
R⁴, R^{4a}, R^{4b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R⁵, which are the same or different;
R⁵ is halogen; CN; OR⁶; SR⁶; N(R⁶R^{6a}); or NO₂;
R⁶, R^{6a} are independently selected from the group consisting of H; and C₁₋₄ alkyl, wherein C₁₋₄ alkyl is optionally substituted with one or more halogen, which are the same or different;
m is 0; 1; or 2;
n is 0; 1; or 2;
**characterized in that** the compound of formula (I) is immobilized on a solid support,
under conditions allowing the formation of a complex between PARP and the immobilization product, and
c) separating PARP from the immobilization product.

9. The method of any of claims 2 to 8, wherein the provision of a protein preparation includes the steps of harvesting at least one cell containing PARP and lysing the cell.

10. The method of any of claims 2 to 9, wherein the steps of the formation of the complex are performed under essentially physiological conditions.

11. A method for determining the presence of PARP in a sample, comprising the steps of:
a) providing a protein preparation expected to contain PARP,
b) contacting the protein preparation with an immobilization product comprising a compound of formula (I) 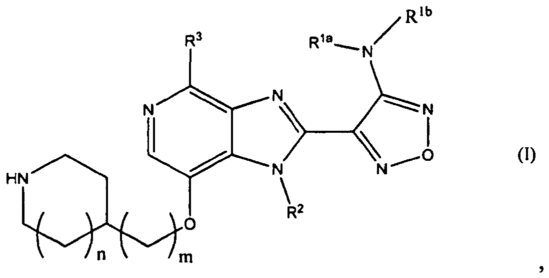 or a salt thereof, wherein
R^{1a}, R^{1b}, R² are independently selected from the group consisting of H; and C₁₋₄ alkyl, wherein C₁₋₄ alkyl is optionally substituted with one or more halogen, which are the same or different;
R³ is H; halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R⁵, which are the same or different;
R⁴, R^{4a}, R^{4b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R⁵, which are the same or different;
R⁵ is halogen; CN; OR⁶; SR⁶; N(R⁶R^{6a}); or NO₂;
R⁶, R^{6a} are independently selected from the group consisting of H; and C₁₋₄ alkyl, wherein C₁₋₄ alkyl is optionally substituted with one or more halogen, which are the same or different;
m is 0; 1 ; or 2;
n is 0; 1; or 2;
**characterized in that** the compound of formula (I) is immobilized on a solid support,
under conditions allowing the formation of a complex between PARP and the immobilization product, and
c) detecting whether PARP has formed a complex with the immobilization product.

12. The method of any of claims 1 to 11, wherein the compound of formula (I) is selected from the group consisting of

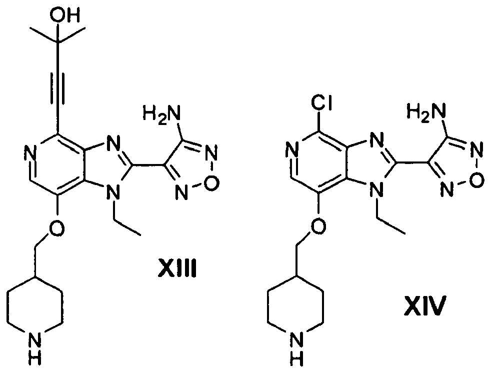

13. The method of any of claims 1 to 12, wherein the solid support is selected from the group consisting of agarose, modified agarose, sepharose beads (e.g. NHS-activated sepharose), latex, cellulose, and ferro- or ferrimagnetic particles.

14. The method of any of claims 1 to 13, wherein the immobilization product results from a covalent direct or linker mediated attachment of the immobilization compound to the solid support, in particular wherein the linker is a C₁₋₁₀ alkylene group, which is optionally interrupted by one or more atoms or functional groups selected from the group consisting of S, O, NH, C(O)O, C(O), and C(O)NH and wherein the linker is optionally substituted with one or more substituents independently selected from the group consisting of halogen, OH, NH₂, C(O)H, C(O)NH₂, SO₃H, NO₂, and CN, in particular wherein said immobilization occurs via the ring nitrogen atom of the saturated ring in formula (I) of claim 1.

15. Use of an immobilization product comprising a compound of formula (I) 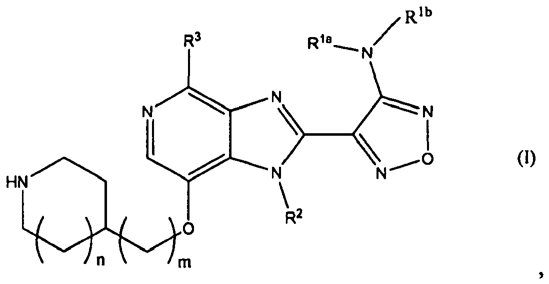 or a salt thereof, wherein
R^{1a}, R^{1b}, R² are independently selected from the group consisting of H; and C₁₋₄ alkyl, wherein C₁₋₄ alkyl is optionally substituted with one or more halogen, which are the same or different;
R³ is H; halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R⁵, which are the same or different;
R⁴, R^{4a}, R^{4b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R⁵, which are the same or different;
R⁵ is halogen; CN; OR⁶; SR⁶; N(R⁶R^{6a}); or NO₂;
R⁶, R^{6a} are independently selected from the group consisting of H; and C₁₋₄ alkyl, wherein C₁₋₄ alkyl is optionally substituted with one or more halogen, which are the same or different;
m is 0; 1 ; for 2;
n is 0; 1; or 2;
**characterized in that** the compound of formula (I) is immobilized on a solid support, for the identification of PARP interacting compounds or for the purification of PARP.

16. Use according to claim 15, wherein the immobilization product is characterized as defined in any of claims 12 to 14.

## Patentansprüche

1. Verfahren für die Identifizierung einer PARP-interagierenden Verbindung, umfassend die Schritte
a) Bereitstellen einer Proteinzubereitung enthaltend PARP,
b) Inkontaktbringen der Proteinzubereitung mit einem Immobilisierungsprodukt, umfassend eine Verbindung der Formel (I) 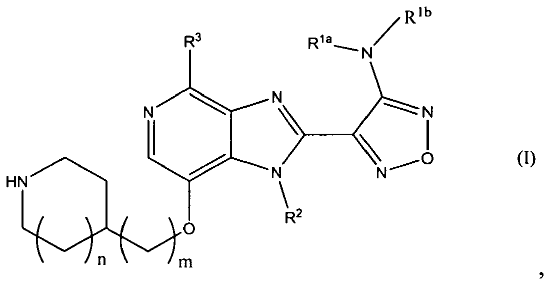 oder eines Salzes davon, wobei
R^{1a}, R^{1b}, R² unabhängig voneinander ausgewählt sind aus der Gruppe bestehend aus H; und C₁₋₄ Alkyl, wobei C₁₋₄ Alkyl wahlweise substituiert ist mit einem oder mehreren Halogenen, die gleich oder unterschiedlich sind;
R³ ist H; Halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ Alkyl; C₂₋₆ Alkenyl; oder C₂₋₆ Alkinyl, wobei C₁₋₆ Alkyl; C₂₋₆ Alkenyl; und C₂₋₆ Alkinyl wahlweise substituiert sind mit einem oder mehreren R⁵, die gleich oder unterschiedlich sind;
R⁴, R^{4a} , R^{4b} unabhängig voneinander ausgewählt sind aus der Gruppe bestehend aus H; C₁₋₆ Alkyl; C₂₋₆ Alkenyl; und C₂₋₆ Alkinyl, wobei C₁₋₆ Alkyl;
C₂₋₆ Alkenyl; und C₂₋₆ Alkinyl wahlweise substituiert sind mit einem oder mehreren R⁵, die gleich oder unterschiedlich sind;
R⁵ ist Halogen; CN; OR⁶; SR⁶; N(R⁶R^{6a}); oder NO₂;
R⁶, R^{6a} unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
m ist 0; 1; oder 2;
n ist 0; 1; oder 2;
**dadurch gekennzeichnet, dass** die Verbindung der Formel (I) auf einem festen Träger immobilisiert ist,
und mit einer gegebenen Verbindung unter Bedingungen inkubiert wird, die die Ausbildung eines Komplexes zwischen PARP und dem Immobilisierungsprodukt erlauben, und
c) Nachweisen des Komplexes, der in Schritt b) ausgebildet wird.

2. Verfahren für die Identifizierung einer PARP-interagierenden Verbindung, umfassend die Schritte:
a) Bereitstellen zweier Aliquots einer Proteinzubereitung enthaltend PARP,
b) Inkontaktbringen eines Aliquots mit einem Immobilisierungsprodukt, umfassend eine Verbindung der Formel (I) 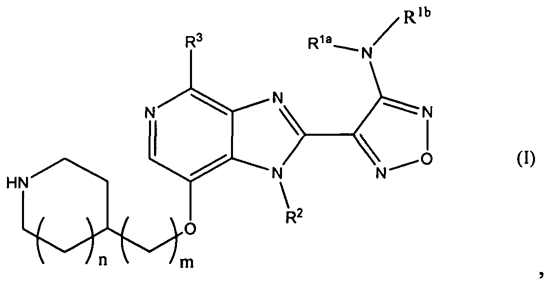 oder eines Salzes davon, wobei
R^{1a}, R^{1b} , R² unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
R³ ist H; Halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R₄; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ Alkyl; C₂₋₆ Alkenyl; oder C₂₋₆ Alkinyl, wobei C₁₋₆ Alkyl; C₂₋₆ Alkenyl; und C₂₋₆ Alkinyl wahlweise substituiert sind mit einem oder mehreren R⁵, die gleich oder unterschiedlich sind;
R⁴, R^{4a}, R^{4b} unabhängig voneinander ausgewählt sind aus der Gruppe bestehend aus H; C₁₋₆ Alkyl; C₂₋₆ Alkenyl; und C₂₋₆ Alkinyl, wobei C₁₋₆ Alkyl; C₂₋₆ Alkenyl; und C₂₋₆ Alkinyl wahlweise substituiert sind mit einem oder mehreren R⁵, die gleich oder unterschiedlich sind;
R⁵ ist Halogen; CN; OR⁶; SR⁶; N(R⁶R^{6a}); oder NO₂;
R⁶, R^{6a} unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
m ist 0; 1; oder 2;
n ist 0; 1; oder 2;
**dadurch gekennzeichnet, dass** die Verbindung der Formel (I) auf einem festen Träger immobilisiert ist,
unter Bedingungen, die die Ausbildung eines Komplexes zwischen PARP und dem Immobilisierungsprodukt erlauben,
c) Inkontaktbringen des anderen Aliquots mit dem Immobilisierungsprodukt und mit einer gegebenen Verbindung unter Bedingungen, die die Ausbildung des Komplexes erlauben, und
d) Bestimmen der Menge des Komplexes, die in den Schritten b) und c) ausgebildet wird.

3. Verfahren für die Identifizierung einer PARP-interagierenden Verbindung, umfassend die Schritte:
a) Bereitstellen zweier Aliquots umfassend jeweils mindestens eine Zelle enthaltend PARP,
b) Inkubieren eines Aliquots mit einer gegebenen Verbindung,
c) Ernten der Zellen eines jeden Aliquots,
d) Lysieren der Zellen, um Proteinzubereitungen zu erhalten,
e) Inkontaktbringen der Proteinzubereitungen mit einem Immobilisierungsprodukt, umfassend eine Verbindung der Formel (I) 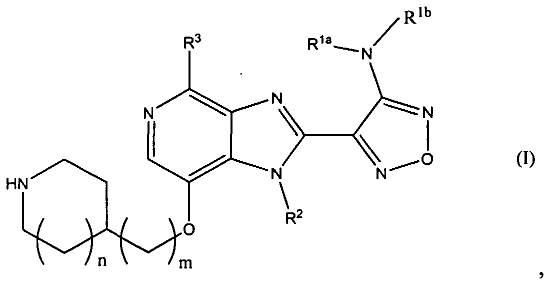 oder eines Salzes davon, wobei
R^{1a}, R^{1b}, R² unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
R³ ist H; Halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ Alkyl; C₂₋₆ Alkenyl; oder C₂₋₆ Alkinyl, wobei C₁₋₆ Alkyl; C₂₋₆ Alkenyl; und C₂₋₆ Alkinyl wahlweise substituiert sind mit einem oder mehreren R⁵, die gleich oder unterschiedlich sind;
R⁴, R^{4a}, R^{4b} unabhängig voneinander ausgewählt sind aus der Gruppe bestehend aus H; C₁₋₆ Alkyl; C₂₋₆ Alkenyl; und C₂₋₆ Alkinyl, wobei C₁₋₆ Alkyl; C₂₋₆ Alkenyl; und C₂₋₆ Alkinyl wahlweise substituiert sind mit einem oder mehreren R⁵, die gleich oder unterschiedlich sind;
R⁵ ist Halogen; CN; OR⁶; SR⁶; N(R⁶R^{6a}); oder NO₂;
R⁶, R^{6a} unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
m ist 0; 1; oder 2;
n ist 0; 1; oder 2;
**gekennzeichnet dadurch, dass** die Verbindung der Formel (I) auf einem festen Träger immobilisiert ist,
unter Bedingungen, die die Ausbildung eines Komplexes zwischen PARP und dem Immobilisierungsprodukt erlauben, und
f) Bestimmen der Menge des Komplexes, die in jedem Aliquot in Schritt e) ausgebildet wird.

4. Verfahren nach einem beliebigen der Ansprüche 2 oder 3, wobei eine reduzierte Menge des Komplexes, die sich in dem mit der Verbindung inkubierten Aliquot im Vergleich zu dem nicht mit der Verbindung inkubierten Aliquot ausgebildet hat, darauf hinweist, dass PARP ein Ziel dieser Verbindung ist.

5. Verfahren nach einem beliebigen der Ansprüche 2 bis 4, wobei die Menge des Komplexes bestimmt wird durch Abtrennen von PARP von dem Immobilisierungsprodukt und anschließendem Nachweis der abgetrennten PARP oder anschließender Bestimmung der Menge an abgetrennter PARP, insbesondere wobei PARP nachgewiesen wird oder die Menge an PARP mittels Massenspektrometrie oder Immundetektionsverfahren bestimmt wird, vorzugsweise mit einem Antikörper, der gegen PARP gerichtet ist.

6. Verfahren nach einem beliebigen der Ansprüche 2 bis 5, wobei die gegebene Verbindung ausgewählt ist aus der Gruppe bestehend aus synthetischen Verbindungen oder organisch-synthetischen Wirkstoffen, bevorzugt niedermolekularen organischen Wirkstoffen und natürlichen niedermolekularen Verbindungen.

7. Verfahren nach einem beliebigen der Ansprüche 2 bis 6, wobei die gegebene Verbindung ein PARP-Inhibitor ist.

8. Verfahren für die Aufreinigung von PARP, umfassend die Schritte
a) Bereitstellen einer Proteinzubereitung enthaltend PARP,
b) Inkontaktbringen der Proteinzubereitung mit einem Immobilisierungsprodukt, umfassend eine Verbindung der Formel (I) 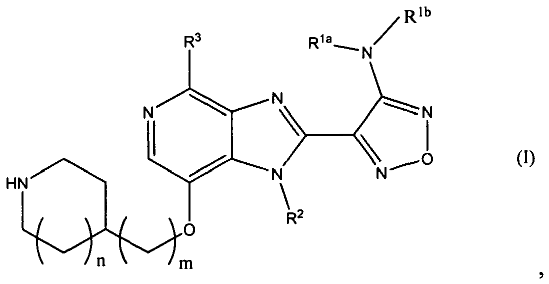 oder eines Salzes davon, wobei
R^{1a}, R^{1b}, R² unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
R³ ist H; Halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ Alkyl; C₂₋₆ Alkenyl; oder C₂₋₆ Alkinyl, wobei C₁₋₆ Alkyl; C₂₋₆ Alkenyl; und C₂₋₆ Alkinyl wahlweise mit einem oder mehreren R⁵ substituiert sind, die gleich oder unterschiedlich sind;
R^{a}, R^{4a} , R^{4b} unabhängig voneinander ausgewählt sind aus der Gruppe bestehend aus H; C₁₋₆ Alkyl; C₂₋₆ Alkenyl; und C₂₋₆ Alkinyl, wobei C₁₋₆ Alkyl; C₂₋₆ Alkenyl; und C₂₋₆ Alkinyl wahlweise substituiert sind mit einem oder mehreren R⁵, die gleich oder unterschiedlich sind;
R⁵ ist Halogen; CN; OR⁶; SR⁶; N(R⁶R^{6a}); oder NO₂;
R⁶, R^{6a} unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
m ist 0; 1; oder 2;
n ist 0; 1; oder 2;
**dadurch gekennzeichnet, dass** die Verbindung der Formel (I) auf einem festen Träger immobilisiert ist,
unter Bedingungen, die die Ausbildung eines Komplexes zwischen PARP und dem Immobilisierungsprodukt erlauben, und
c) Abtrennen von PARP von dem Immobilisierungsprodukt.

9. Verfahren nach einem beliebigen der Ansprüche 2 bis 8, wobei die Bereitstellung einer Proteinzubereitung die Schritte des Erntens mindestens einer Zelle, die PARP enthält, und Lysieren der Zelle einschließt.

10. Verfahren nach einem beliebigen der Ansprüch 2 bis 9, wobei die Schritte der Ausbildung des Komplexes unter im Wesentlichen physiologischen Bedingungen durchgeführt werden.

11. Verfahren zum Bestimmen der Anwesenheit von PARP in einer Probe, umfassend die Schritte:
a) Bereitstellen einer Proteinzubereitung, von der erwartet wird, dass sie PARP enthält,
b) Inkontaktbringen der Proteinzubereitung mit einem Immobilisierungsprodukt, umfassend eine Verbindung der Formel (I) 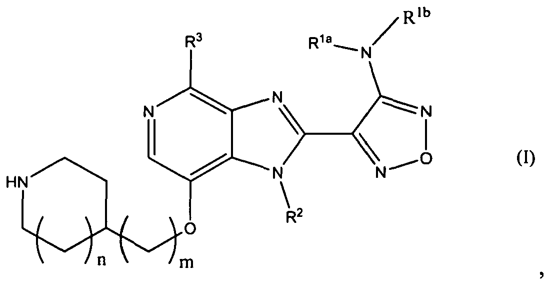 oder eines Salzes davon, wobei
R^{1a}, R^{1b}, R² unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
R³ ist H; Halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆, Alkyl; C₂₋₆ Alkenyl; oder C₂₋₆ Alkinyl, wobei C₁₋₆ Alkyl; C₂₋₆ Alkenyl; und C₂₋₆ Alkinyl wahlweise substituiert sind mit einem oder mehreren R⁵, die gleich oder unterschiedlich sind;
R⁴, R^{4a}, R^{4b} unabhängig voneinander ausgewählt sind aus der Gruppe bestehend aus H; C₁₋₆ Alkyl; C₂₋₆ Alkenyl; und C₂₋₆ Alkinyl, wobei C₁₋₆ Alkyl;
C₂₋₆ Alkenyl; und C₂₋₆ Alkinyl wahlweise substituiert sind mit einem oder mehreren R⁵, die gleich oder unterschiedlich sind;
R⁵ ist Halogen; CN; OR⁶; SR⁶; N(R⁶R^{6a}); oder NO₂;
R⁶, R^{6a} unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
m ist 0; 1; oder 2;
n ist 0; 1; oder 2;
**dadurch gekennzeichnet, dass** die Verbindung der Formel (I) auf einem festen Träger immobilisiert ist,
unter Bedingungen, die die Ausbildung eines Komplexes zwisschen PARP und dem Immobilisierungsprodukt erlauben, und
c) Nachweisen, ob PARP einen Komplex mit dem Immobilisierungsprodukt ausgebildet hat.

12. Verfahren nach einem beliebigen der Ansprüche 1 bis 11, wobei die Verbindung der Formel (I) ausgewählt ist aus der Gruppe bestehend aus

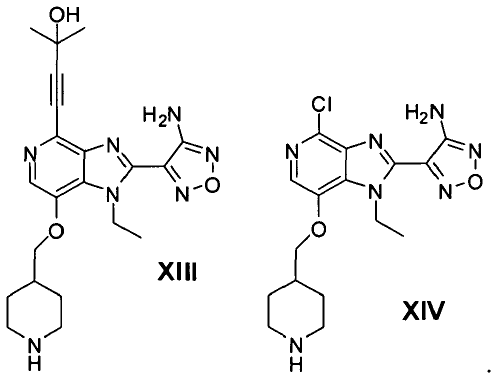

13. Verfahren nach einem beliebigen der Ansprüche 1 bis 12, wobei der feste Träger ausgewählt ist aus der Gruppe bestehend aus Agarose, modifizierter Agarose, Sepharose-Harzkömem (z.B. NHS-aktivierter Sepharose), Latex, Zellulose und ferro- oder ferrimagnetischen Partikeln.

14. Verfahren nach einem beliebigen der Ansprüche 1 bis 13, wobei das Immobilisierungsprodukt resultiert aus einer kovalenten direkten oder Linkervermittelten Verknüpfung der Immobilisierungsverbindung an den festen Träger, insbesondere wobei der Linker eine C₁₋₁₀ Alkylengruppe ist, die wahlweise durch ein oder mehrere Atome oder funktionelle Gruppen, ausgewählt aus der Gruppe bestehend aus S, O, NH, C(O)O, C(O) und C(O)NH, unterbrochen ist und wobei der Linker wahlweise substituiert ist mit einem oder mehreren Substituenten, die unabhängig voneinander ausgewählt sind aus der Gruppe bestehend aus Halogen, OH, NH₂, C(O)H, C(O)NH₂, SO₃H, NO₂ und CN, insbesondere wobei die Immobilisierung durch das Ringstickstoffatom des gesättigten Rings in Formel I nach Anspruch 1 erfolgt.

15. Verwendung eines Immobilisierungsproduktes, umfassend eine Verbindung der Formel (I) 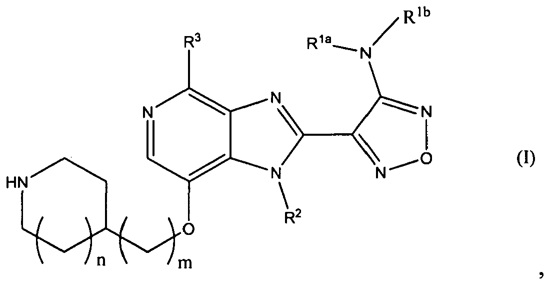 oder eines Salzes davon, wobei
R^{1a}, R^{1b}, R² unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
R³ ist H; Halogen; CN; C(O)OR⁴; OR⁴; C(O)R⁴; C(O)N(R⁴R^{4a}); S(O)₂N(R⁴R^{4a}); S(O)N(R⁴R^{4a}); S(O)₂R⁴; S(O)R⁴; SR⁴; N(R⁴R^{4a}); NO₂; OC(O)R⁴; N(R⁴)C(O)R^{4a}; N(R⁴)S(O)₂R^{4a}; N(R⁴)S(O)R^{4a}; N(R⁴)C(O)N(R^{4a}R^{4b}); N(R⁴)C(O)OR^{4a}; OC(O)N(R⁴R^{4a}); C₁₋₆ Alkyl; C₂₋₆ Alkenyl; oder C₂₋₆ Alkinyl, wobei C₁₋₆ Alkyl; C₂₋₆ Alkenyl; und C₂₋₆ Alkinyl wahlweise substituiert sind mit einem oder mehreren R⁵, die gleich oder unterschiedlich sind;
R⁴, R^{4a}, R^{ab} unabhängig voneinander ausgewählt sind aus der Gruppe bestehend aus H; C₁₋₆ Alkyl; C₂₋₆ Alkenyl; und C₂₋₆ Alkinyl, wobei C₁₋₆ Alkyl; C₂₋₆ Alkenyl; und C₂₋₆ Alkinyl wahlweise substituiert sind mit einem oder mehreren R⁵, die gleich oder unterschiedlich sind;
R⁵ ist Halogen; CN; OR⁶; SR⁶; N(R⁶R^{6a}); oder NO₂;
R⁶, R^{6a} unabhängig voneinander ausgewählt sind aus der Gruppe bestehend aus H; und C₁₋₄ Alkyl, wobei C₁₋₄ Alkyl wahlweise substituiert ist mit einem oder mehreren Halogen(en), die gleich oder unterschiedlich sind;
m ist 0; 1; oder 2;
n ist 0; 1; oder 2;
**dadurch gekennzeichnet, dass** die Verbindung der Formel (I) auf einem festen Träger immobilisiert ist, für die Identifikation von PARP-interagierenden Verbindungen oder für die Aufreinigung von PARP.

16. Verwendung gemäß Anspruch 15, wobei das Immobilisierungsprodukt gekennzeichnet ist wie in einem beliebigen der Ansprüche 12 bis 14 definiert.

## Revendications

1. Procédé pour l'identification d'un composé interagissant avec PARP, comprenant les étapes de
a) fournir une préparation protéique contenant PARP,
b) mettre en contact la préparation protéique avec un produit d'immobilisation comprenant un composé de formule (I) 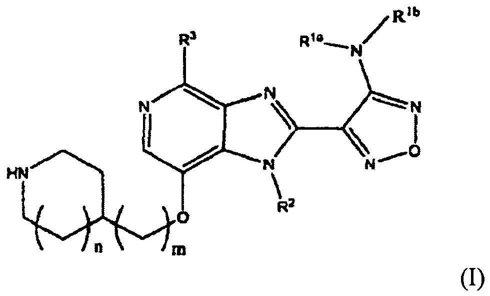 ou un sel de celui-ci, dans lequel
R^{1a}, R^{1b}, R² sont indépendamment choisis dans le groupe constitué de H ; et un alkyle en C₁₋₄, dans lequel l'alkyle en C₁₋₄ est optionnellement substitué par un ou plusieurs halogènes, qui sont identiques ou différents ;
R³ est un H ; un halogène ; un CN ; C(O)OR⁴ ; OR⁴ ; C(O) R⁴, C(O)N(R⁴R^{4a}) ; S(O)₂N(R⁴R^{4a}); S(O)N(R⁴R^{4a}) ; S(O)2R⁴ ; S(O)R⁴ ; SR⁴ ; N(R4R^{4a}); NO₂ ; OC(O)R⁴ ; N(R⁴)C(O)R^{4a} ; N(R⁴)S(O)₂R^{4a} ; N(R⁴)S(O)R^{4a} ; N(R⁴)C(O)N(R^{4a}R^{4b}) ; N(R⁴)C(O)OR^{4a} ; OC(O)N(R⁴ R^{4a}) ; un alkyle en C₁₋₆ ; un alcényle en C₂₋₆ ; ou un alcynyle en C₂₋₆ ; dans lequel un alkyle en C₁₋₆, un alcényle en C₂₋₆ et un alcynyle en C₂₋₆ sont optionnellement substitués par un ou plusieurs R⁵, qui sont identiques ou différents ;
R⁴, R^{4a}, R^{4b} sont indépendamment choisis dans le groupe constitué de H ; un alkyle en C₁₋₆ ; un alcényle en C₂₋₆ et un alcynyle en C₂₋₆ dans lequel un alkyle en C₁₋₆ ; un alcényle en C₂₋₆ et un alcynyle en C₂₋₆ sont optionnellement substitués par un ou plusieurs R⁵, qui sont identiques ou différents ;
R⁵ est un halogène ; CN ; OR⁶ ; SR⁶ ; N(R⁶R^{6a}) ou NO₂ ;
R6, R6a sont indépendamment choisis dans le groupe constitué de H ; et un alkyle en C1-4, dans lequel l'alkyle en C1-4 est optionnellement substitué par un ou plusieurs halogènes, qui sont identiques ou différents ;
m est égal à 0, 1 ou 2 ;
n est égal à 0, 1 ou 2 ;
**caractérisé en ce que** le composé de formule (I) est immobilisé sur un support solide,
et avec un composé donné dans des conditions permettant la formation d'un complexe entre PARP et le produit d'immobilisation, et
c) détecter le complexe formé dans l'étape b).

2. Procédé pour l'identification d'un composé interagissant avec PARP, comprenant les étapes de :
a) fournir deux aliquotes d'une préparation protéique contenant PARP,
b) mettre en contact une aliquote avec un produit d'immobilisation comprenant un composé de formule (I) 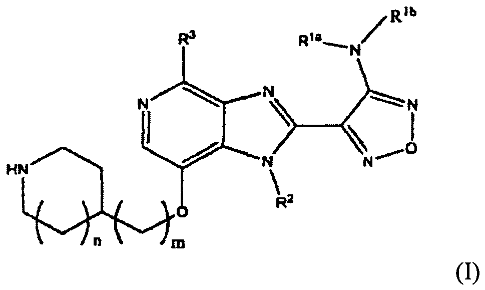 ou un sel de celui-ci, dans lequel
R^{1a}, R^{1b}, R² sont indépendamment choisis dans le groupe constitué de H ; et un alkyle en C₁₋₄, dans lequel l'alkyle en C₁₋₄ est optionnellement substitué par un ou plusieurs halogènes, qui sont identiques ou différents ;
R³ est un H ; un halogène ; un CN ; C(O)OR⁴ ; OR⁴ ; C(O) R⁴, C(O)N(R⁴R^{4a}) ; S(O)N(R⁴R^{4a}); S(O)N(R⁴R^{4a}) S(O)₂R⁴ ; S(O)R⁴ ; SR⁴ ; N(R⁴R^{4a}) ; NO₂ ; OC(O)R⁴ ; N(R⁴)C(O)R^{4a} ; N(R⁴)S(O)₂R^{4a} ; N(R)S(O)R^{4a}; N(R⁴)C(O)N(R^{4a}R^{4b}) ; N(R⁴)C(O)OR^{4a} ; OC(O)N(R⁴ R^{4a}) ; un alkyle en C₁₋₆ ; un alcényle en C₂₋₆ ; ou un alcynyle en C₂₋₆; dans lequel un alkyle en C₁₋₆, un alcényle en C₂₋₆ et un alcynyle en C₂₋₆ sont optionnellement substitués par un ou plusieurs R⁵, qui sont identiques ou différents ;
R⁴, R^{4a}, R^{4b} sont indépendamment choisis dans le groupe constitué de H ; un alkyle en C₁₋₆ ; un alcényle en C₂₋₆ et un alcynyle en C₂₋₆ dans lequel un alkyle en C₁₋₆ ; un alcényle en C₂₋₆ et un alcynyle en C₂₋₆ sont optionnellement substitués par un ou plusieurs R⁵, qui sont identiques ou différents ;
R⁵ est un halogène ; CN ; OR⁶ ; SR⁶ ; N(R⁶R^{6a}) ou NO₂ ;
R⁶, R^{6a} sont indépendamment choisis dans le groupe constitué de H ; et un alkyle en C₁₋₄, dans lequel l'alkyle en C₁₋₄ est optionnellement substitué par un ou plusieurs halogènes, qui sont identiques ou différents ;
m est égal à 0, 1 ou 2 ;
n est égal à 0, 1 ou 2 ;
**caractérisé en ce que** le composé de formule (I) est immobilisé sur un support solide,
dans des conditions permettant la formation d'un complexe entre PARP et le produit d'immobilisation,
c) la mise en contact de l'autre aliquote avec le produit d'immobilisation et avec un composé donné dans des conditions permettant la formation du complexe, et
d) la détermination de la quantité de complexe formé dans les étapes b) et c).

3. Procédé pour l'identification d'un composé d'interaction avec PARP, comprenant les étapes de :
a) fournir deux aliquotes comprenant chacune au moins une cellule contenant PARP,
b) incuber une aliquote avec un composé donné,
c) récolter les cellules de chaque aliquote,
d) lyser les cellules, afin d'obtenir des préparations protéiques,
e) mettre en contact les préparations protéiques avec un produit d'immobilisation comprenant un composé de formule (I) 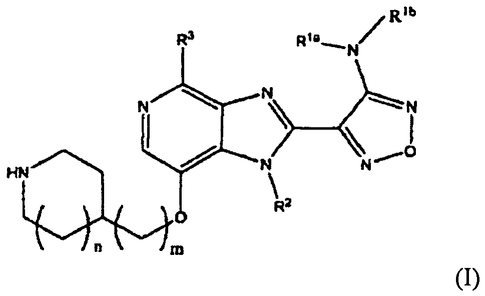 ou un sel de celui-ci, dans lequel
R^{1a}, R^{1b}, R² sont indépendamment choisis dans le groupe constitué de H ; et un alkyle en C₁₋₄, dans lequel l'alkyle en C₁₋₄ est optionnellement substitué par un ou plusieurs halogènes, qui sont identiques ou différents ;
R³ est un H ; un halogène ; un CN ; C(O)OR⁴ ; OR⁴ ; C(O) R⁴, C(O)N(R⁴R^{4a}) ; S(O)₂N(R^{a}R^{4a}) ; S(O)N(R⁴R^{4a}) ; S(O)₂R⁴; S(O)R⁴; SR⁴ ; N(R⁴R^{4a}) ; NO₂ ; OC(O)R⁴ ; N(R⁴)C(O)R^{4a} ; N(R⁴)S(O)₂R^{4a} ; N(R⁴)S(O)R^{4a} ; N(R⁴)C(O)N(R^{4a}R^{4b}) ; N(R⁴)C(O)OR^{4a} ; OC(O)N(R⁴ R^{4a}) ; un alkyle en C₁₋₆ ; un alcényle en C₂₋₆ ; ou un alcynyle en C₂₋₆; dans lequel un alkyle en C₁₋₆, un alcényle en C₂₋₆ et un alcynyle en C₂₋₆ sont optionnellement substitués par un ou plusieurs R⁵, qui sont identiques ou différents ;
R⁴, R^{4a}, R^{4b} sont indépendamment choisis dans le groupe constitué de H ; un alkyle en C₁₋₆ ; un alcényle en C₂₋₆ et un alcynyle en C₂₋₆ dans lequel un alkyle en C₁₋₆ ; un alcényle en C₂₋₆ et un alcynyle en C₂₋₆ sont optionnellement substitués par un ou plusieurs R⁵, qui sont identiques ou différents ;
R⁵ est un halogène ; CN ; OR⁶ ; SR⁶ ; N(R⁶R^{6a}) ou NO₂ ;
R⁶, R^{6a} sont indépendamment choisis dans le groupe constitué de H ; et un alkyle en C₁₋₄, dans lequel l'alkyle en C₁₋₄ est optionnellement substitué par un ou plusieurs halogènes, qui sont identiques ou différents ;
m est égal à 0, 1 ou 2 ;
n est égal à 0, 1 ou 2 ;
**caractérisé en ce que** le composé de formule (I) est immobilisé sur un support solide,
dans des conditions permettant la formation d'un complexe entre PARP et le produit d'immobilisation, et
f) déterminer la quantité du complexe formé dans chaque aliquote dans l'étape e).

4. Procédé selon l'une quelconque des revendications 2 ou 3, dans lequel une quantité réduite du complexe formé dans l'aliquote incubée avec le composé, en comparaison avec l'aliquote non incubée avec le composé, indique que PARP est une cible du composé.

5. Procédé selon l'une quelconque des revendications 2 à 4, dans lequel la quantité du complexe est déterminée en séparant PARP du produit d'immobilisation et en détectant successivement le PARP séparé ou en déterminant successivement la quantité de PARP séparé, en particulier dans lequel PARP est détecté ou la quantité de PARP est déterminée par spectrométrie de masse, ou des procédés d'immuno-détection, de préférence avec un anticorps dirigé contre PARP.

6. Procédé selon l'une quelconque des revendications 2 à 5, dans lequel ledit composé donné est choisi à partir du groupe constitué de composés synthétiques, ou de médicaments synthétiques organiques, de préférence des médicaments organiques à petites molécules, et des composés à petites molécules naturels.

7. Procédé selon l'une quelconque des revendications 2 à 6, dans lequel le composé donné est un inhibiteur de PARP.

8. Procédé de purification de PARP, comprenant les étapes de :
a) fournir une préparation protéique contenant PARP,
b) mettre en contact la préparation protéique avec un produit d'immobilisation comprenant un composé de formule (I) 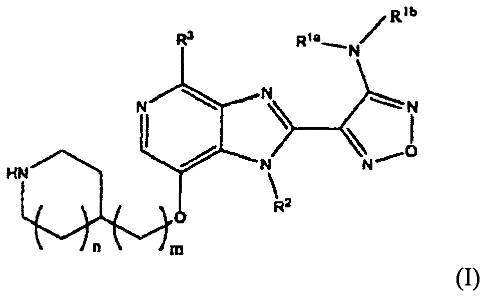 ou un sel de celui-ci, dans lequel
R^{1a}, R^{1b}, R² sont indépendamment choisis dans le groupe constitué de H ; et un alkyle en C₁₋₄, dans lequel l'alkyle en C₁₋₄ est optionnellement substitué par un ou plusieurs halogènes, qui sont identiques ou différents ;
R³ est un H ; un halogène ; un CN ; C(O)OR⁴ ; OR⁴ ; C(O) R⁴, C(O)N(R⁴R^{4a}) ; S(O)₂N(R⁴R^{4a}) ; S(O)N(R⁴R^{4a}) ; S(O)₂R⁴ ; S(O)R⁴ ; SR⁴ ; N(R⁴R^{4a}) ; NO₂ ; OC(O)R⁴ ; N(R⁴)C(O)R^{4a} ; N(R⁴)S(O)₂R^{4a} ; N(R⁴)S(O)R^{4a}; N(R⁴)C(O)N(R^{4a}R^{4b}) ; N(R⁴)C(O)OR^{4a} ; OC(O)N(R⁴ R^{4a}) ; un alkyle en C₁₋₆ ; un alcényle en C₂₋₆ ; ou un alcynyle en C₂₋₆; dans lequel un alkyle en C₁₋₆, un alcényle en C₂₋₆ et un alcynyle en C₂₋₆ sont optionnellement substitués par un ou plusieurs R⁵, qui sont identiques ou différents ;
R^{a}, R^{4a}, R^{4b} sont indépendamment choisis dans le groupe constitué de H ; un alkyle en C₁₋₆ ; un alcényle en C₂₋₆ et un alcynyle en C₂₋₆ dans lequel un alkyle en C₁₋₆ ; un alcényle en C₂₋₆ et un alcynyle en C₂₋₆ sont optionnellement substitués par un ou plusieurs R⁵, qui sont identiques ou différents ;
R⁵ est un halogène ; CN ; OR⁶ ; SR⁶ ; N(R⁶R^{6a}) ou NO₂ ;
R⁶, R^{6a} sont indépendamment choisis dans le groupe constitué de H ; et un alkyle en C₁₋₄, dans lequel l'alkyle en C₁₋₄ est optionnellement substitué par un ou plusieurs halogènes, qui sont identiques ou différents ;
m est égal à 0, 1 ou 2 ;
n est égal à 0, 1 ou 2 ;
**caractérisé en ce que** le composé de formule (I) est immobilisé sur un support solide,
dans des conditions permettant la formation d'un complexe entre PARP et le produit d'immobilisation, et
c) séparer PARP du produit d'immobilisation.

9. Procédé selon l'une quelconque des revendications 2 à 8, dans lequel la fourniture d'une préparation protéique comprend les étapes de récolte d'au moins une cellule contenant du PARP et de lyse de la cellule.

10. Procédé selon l'une quelconque des revendications 2 à 9, dans lequel les étapes de la formation du complexe sont réalisées dans des conditions essentiellement physiologiques.

11. Procédé de détermination de la présence de PARP dans un échantillon, comprenant les étapes de :
a) fournir une préparation protéique supposée contenir PARP,
b) mettre en contact la préparation protéique avec un produit d'immobilisation comprenant un composé de formule (I) 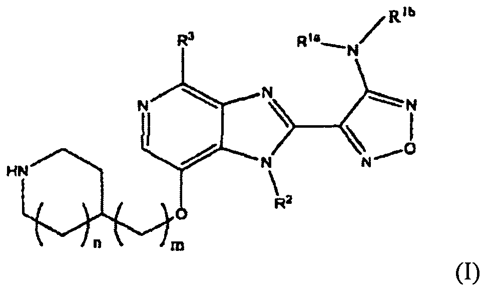 ou un sel de celui-ci, dans lequel
R^{1a}, R^{1b}, R² sont indépendamment choisis dans le groupe constitué de H ; et un alkyle en C1-4, dans lequel l'alkyle en C1-4 est optionnellement substitué par un ou plusieurs halogènes, qui sont identiques ou différents ;
R3 est un H; un halogène; un CN; C(O)OR⁴ ; OR4; C(O) R4, C(O)N(R4R4a) ; S(O)2N(R4R4a) ; S(O)N(R4R4a) ; S(O)2R4 ; S(O)R4 ; SR4 ; N(R4R4a) ; N02 ; OC(O)R4 ; N(R⁴)C(O)R^{4a} ; N(R⁴)S(O)₂R^{4a} ; N(R⁴)S(O)R^{4a} ; N(R⁴)C(O)N(R^{4a}R⁴b) ; N(R⁴)C(O)OR^{4a} ; OC(O)N(R⁴ R^{4a}) ; un alkyle en C₁₋₆ ; un alcényle en C₂₋₆ ; ou un alcynyle en C₂₋₆ ; dans lequel un alkyle en C₁₋₆, un alcényle en C₂₋₆ et un alcynyle en C₂₋₆ sont optionnellement substitués par un ou plusieurs R⁵, qui sont identiques ou différents ;
R4, R4a, R4b sont indépendamment choisis dans le groupe constitué de H ; un alkyle en C1-6 ; un alcényle en C2-6 et un alcynyle en C2-6 dans lequel un alkyle en C1-6 ; un alcényle en C2-6 et un alcynyle en C2-6 sont optionnellement substitués par un ou plusieurs R5, qui sont identiques ou différents ;
R5 est un halogène ; CN ; OR6 ; SR6 ; N(R6R6a) ou NO2 ;
R6, R6a sont indépendamment choisis dans le groupe constitué de H ; et un alkyle en C1-4, dans lequel l'alkyle en C1-4 est optionnellement substitué par un ou plusieurs halogènes, qui sont identiques ou différents ;
m est égal à 0, 1 ou 2 ;
n est égal à 0, 1 ou 2 ;
**caractérisé en ce que** le composé de formule (I) est immobilisé sur un support solide,
dans des conditions permettant la formation d'un complexe entre PARP et le produit d'immobilisation, et
c) détecter si PARP a formé un complexe avec le produit d'immobilisation.

12. Procédé selon l'une quelconque des revendications 1 à 11, dans lequel le composé de formule (I) est choisi dans le groupe constitué de

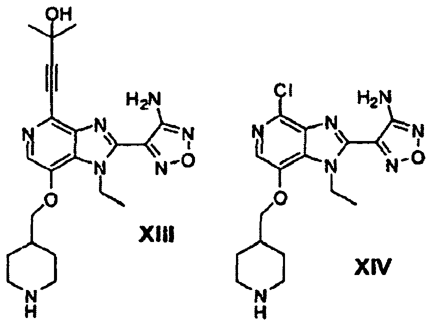

13. Procédé selon l'une quelconque des revendications 1 à 12, dans lequel le support solide est choisi dans le groupe constitué d'agarose, d'agarose modifié, de billes de sépharose (par ex, sépharose activée par NHS), latex, cellulose et particules ferro- ou ferri-magnétiques.

14. Procédé selon l'une quelconque des revendications 1 à 13, dans lequel le produit d'immobilisation résulte d'une fixation directe covalente ou provoquée par un bras de liaison du composé d'immobilisation au support solide, en particulier dans lequel le bras de liaison est un groupe alcylène en C1-10, qui est optionnellement interrompu par un ou plusieurs atomes ou groupes fonctionnels choisis dans le groupe constitué de S, O, NH, C(O)O, C(O) et C(O)NH et dans lequel le bras de liaison est optionnellement substitué par un ou plusieurs substituants indépendamment choisis dans le groupe constitué d'halogène, un OH, NH2, C(O)H, C(O)NH2, SO3H, NO2 et CN, en particulier dans lequel ladite immobilisation a lieu via l'atome d'azote du cycle saturé dans la formule (I) selon la revendication 1.

15. Utilisation d'un produit d'immobilisation comprenant un composé de formule (I) 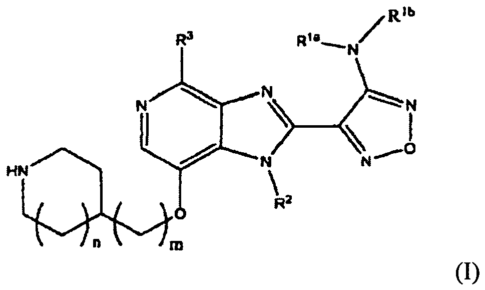 ou un sel de celui-ci, dans lequel
R^{1a}, R^{1b}, R² sont indépendamment choisis dans le groupe constitué de H ; et un alkyle en C₁₋₄, dans lequel l'alkyle en C₁₋₄ est optionnellement substitué par un ou plusieurs halogènes, qui sont identiques ou différents ;
R³ est un H ; un halogène ; un CN ; C(O)OR⁴ ; OR⁴ ; C(O) R⁴, C(O)N(R⁴R^{4a}) ; S(O)₂N(R⁴R^{4a}) ; S(O)N(R⁴R^{4a}) ;S(O)₂R⁴; S(O)R⁴; SR⁴ ; N(R⁴R^{4a}) ; NO₂ ; OC(O)R⁴ ; N(R⁴)C(O)R^{4a}; N(R⁴)S(O)₂R^{4a} ; N(R⁴)S(O)R^{4a} ; N(R⁴)C(O)N(R^{4a}R^{4b}) ; N(R⁴)C(O)OR^{4a} ; OC(O)N(R⁴ R^{4a}) ; un alkyle en C₁₋₆ ; un alcényle en C₂₋₆ ; ou un alcynyle en C₂₋₆; dans lequel un alkyle en C₁₋₆, un alcényle en C₂₋₆ et un alcynyle en C₂₋₆ sont optionnellement substitués par un ou plusieurs R⁵, qui sont identiques ou différents ;
R⁴, R^{4a}, R^{4b} sont indépendamment choisis dans le groupe constitué de H ; un alkyle en C₁₋₆ ; un alcényle en C₂₋₆ et un alcynyle en C₂₋₆ dans lequel un alkyle en C₁₋₆ ; un alcényle en C₂₋₆ et un alcynyle en C₂₋₆ sont optionnellement substitués par un ou plusieurs R⁵, qui sont identiques ou différents ;
R⁵ est un halogène ; CN ; OR⁶ ; SR⁶ ; N(R⁶R^{6a}) ou NO₂ ;
R⁶, R^{6a} sont indépendamment choisis dans le groupe constitué de H ; et un alkyle en C₁₋₄, dans lequel l'alkyle en C₁₋₄ est optionnellement substitué par un ou plusieurs halogènes, qui sont identiques ou différents ;
m est égal à 0, 1 ou 2 ;
n est égal à 0, 1 ou 2 ;
**caractérisé en ce que** le composé de formule (I) est immobilisé sur un support solide,
pour l'identification de composés interagissant avec PARP ou pour la purification de PARP.

16. Utilisation selon la revendication 15, dans laquelle le produit d'immobilisation est caractérisé comme défini dans l'une quelconque des revendications 12 à 14.
